(19) Europäisches Patentamt
European Patent Office
Office européen des brevets

(11) Veröffentlichungsnummer : **0 022 078**
**B1**

(12) # EUROPÄISCHE PATENTSCHRIFT

(45) Veröffentlichungstag der Patentschrift : **25.07.84**

(21) Anmeldenummer : **80810200.8**

(22) Anmeldetag : **16.06.80**

(51) Int. Cl.$^3$ : **C 07 D471/04**, A 61 K 31/47 // (C07D471/04, 231/00, 221/00)

(54) **Pyrazolochinoline, Verfahren zu ihrer Herstellung, und pharmazeutische Präparate enthaltend diese Verbindungen.**

(30) Priorität : **21.06.79 US 50716**

(43) Veröffentlichungstag der Anmeldung : **07.01.81 Patentblatt 81/01**

(45) Bekanntmachung des Hinweises auf die Patenterteilung : **25.07.84 Patentblatt 84/30**

(84) Benannte Vertragsstaaten : **AT BE CH DE FR GB IT LI LU NL SE**

(56) Entgegenhaltungen :
**EP-A- 0 005 745**
**US-A- 3 890 324**
**CHEMISCHES ZENTRALBLATT, 102 Jahrgang, Band I, Nr. 12, 25. März 1931, Berlin R. SEKA et al. "Zur Kenntnis methoxylierter Phenylchinolone bzw. 2-Phenyl-4-oxychinoline" Seiten 1925 bis 1926**
**Die Akte enthält technische Angaben, die nach dem Eingang der Anmeldung eingereicht wurden und die nicht in dieser Patentschrift enthalten sind.**

(73) Patentinhaber : **CIBA-GEIGY AG**
**Postfach**
**CH-4002 Basel (CH)**

(72) Erfinder : **Yokoyama, Naokata**
**200 Winston Drive (Apt. 2013)**
**Cliffside Park New Jersey 07010 (US)**

EP 0 022 078 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

In Monatsh. 57, 52 (1931) sind 2- unsubstituierte 4-Phenyl-pyrazolo [4,3-c] chinolin-3-one und ihre 3-Hydroxy tautomeren Verbindungen beschrieben. Gegenstand der EP-5745 sind antiinflammatorisch, ZNS-depressiv und gegen Angst wirkende 3-Phenyl-pyrazolo [3,4-c] isochinolin-5-one. In J. Chem. Soc. 1959, 599 ist die Synthese von 2-Phenyl-pyrazolo [3,4-c] isochinolin-3-onen (oder 3-olen) und in US-A-3,890,324 sind antiinflammatorisch wirksame 1H-Pyrazolo [4,3-c] chinol-4-[5H] on-3-carbonsäuren offenbart. Es wurde nun überraschend gefunden, dass die 2-Aryl-pyrazolo-[4,3-c] chinolin-3-one wertvolle psychoaktive Wirkstoffe sind.

Die vorliegende Erfindung betrifft 2-Aryl-pyrazolo [4,3-c] chinolin-3-(1 und 5H)-on-Verbindungen der allgemeinen Formeln I und II

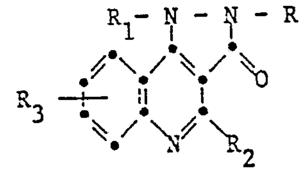 (I) und 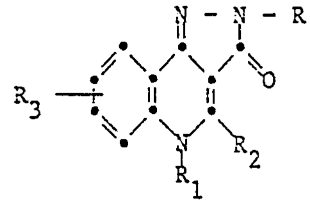 (II)

worin $R_3$ Wasserstoff oder höchstens 3 gleiche oder verschiedene Substituenten der Gruppe $C_1$-$C_7$-Alkyl, $C_1$-$C_7$-Alkoxy, $C_1$-$C_7$-Alkylthio, Hydroxy, Halogen, Trifluormethyl, Nitro, Amino, Mono- oder Di-$C_1$-$C_7$-alkylamino, Cyan, Carbamoyl oder Carboxy bedeutet, R für unsubstituiertes oder durch höchstens 3 der für $R_3$ angegebenen Reste substituiertes Phenyl, Pyridyl, $C_1$-$C_7$-Alkylpyridyl oder Halogenpyridyl steht, $R_1$ Wasserstoff, $C_1$-$C_7$-Alkyl oder (Hydroxy, Di-$C_1$-$C_7$-alkylamino oder $R_3$-Phenyl)-$C_1$-$C_7$-alkyl bedeutet, worin die Hydroxy- oder Aminogruppe vom Ringstickstoffatom durch mindestens 2 Kohlenstoffatome getrennt ist, und $R_2$ für Wasserstoff oder $C_1$-$C_7$-Alkyl steht ; ihre 3-Hydroxy tautomeren Verbindungen ; $C_1$-$C_7$-Alkanoyl-, Carbamoyl-, Mono- oder Di-$C_1$-$C_7$-alkylcarbamoyl-Derivate der genannten (Hydroxy oder Amino)-(phenyl oder phenylen)-Verbindungen ; oder die Salze, insbesondere die pharmazeutisch verwendbaren Salze davon, sowie Verfahren zu ihrer Herstellung, pharmazeutische Präparate enthaltend diese psychoaktiven Verbindungen, sowie die Verbindungen zur therapeutische Verwendung.

Der 1,2-Phenylenrest ist vorzugsweise unsubstituiert oder substituiert durch einen, zwei oder drei Substituenten der Gruppe $C_1$-$C_7$-Alkyl, z. B. Methyl, Aethyl, n- oder i-Propyl oder -Butyl ; $C_1$-$C_7$-Alkoxy, z. B. Methoxy, Aethoxy, n- oder i-Propoxy oder -Butoxy ; $C_1$-$C_7$-Alkylthio, z. B. Methylthio oder Aethylthio ; Hydroxy ; Halogen, z. B. Fluor, Chlor oder Brom ; Trifluormethyl ; Nitro ; Amino ; Mono- oder Di-$C_1$-$C_7$-Alkylamino, z. B. Mono- oder Di-(methyl, äthyl, n- oder i-propyl)-amino ; Cyan, Carbamoyl oder Carboxy.

Der Phenyl- oder Pyridylrest R in 2-Stellung ist vorzugsweise ein ortho-unsubstituierter Phenylrest, der wie der oben definierte 1,2-Phenylrest keinen oder höchstens drei Substituenten aufweist, aber auch 3-, 4- oder 2-Pyridyl-, oder ein $C_1$-$C_7$-alkylierter oder halogenierter Pyridylrest, vorzugsweise (4-Methyl oder 5-Chlor)-2-pyridyl. Von den oben aufgezählten Substituenten des 1,2-Phenylen- und/oder R-Restes ist, beginnend mit Trifluormethyl, jeweils vorzugsweise lediglich ein derartiger Substituent vorhanden.

Das Symbol $R_1$, in entweder 1- oder vorzugsweise 5-Stellung, ist vorzugsweise Wasserstoff, aber auch eine der genannten Alkylgruppen, oder eine (Hydroxy, Di-$C_1$-$C_7$-Alkylamino oder Phenyl-$C_1$-$C_7$-niederalkylgruppe, welche die benachbarten Heteroatome durch mindestens 2 Kohlenstoffatome trennt. Solche Reste sind 2-(Hydroxy, Dimethylamino oder Diäthylamino)-äthyl, 2- oder 3-(Hydroxy oder Dimethylamino)- propyl ; Benzyl 1- oder 2-Phenyläthyl.

Der Rest $R_2$ in 4-Stellung ist auch vorzugsweise Wasserstoff, oder eine der genannten Alkylgruppen, insbesondere Methyl.

Wegen des genannten 1- oder 5-Substituenten $R_1$ sind die Verbindungen der vorliegenden Erfindung richtig als 3-One bzw. als 3-Oxo-Derivate dargestellt. Bedeutet jedoch das Symbol $R_1$ Wasserstoff, so können die Verbindungen, je nach dem Milieu und der Substitution, eine geringe Menge von tautomeren 3-Hydroxy-Verbindungen bilden. Im allgemeinen sind sie jedoch schwache Basen oder Säuren, die Salze entweder mit starken Säuren oder Basen ergeben. Lediglich die genannten (Hydroxy oder Amino)-Phenyl-Verbindungen bilden Acylderivate. Die genannten Acylderivate sind $C_1$-$C_7$-Alkanoyl-, Carbamoyl-, Mono- oder Di-$C_1$-$C_7$-Alkyl-carbamoyl-Derivate der oben genannten (Hydroxy oder Amino)-phenylverbindungen oder (Hydroxy oder Amino)-phenylenverbindungen. Solche Derivate sind z. B. Acetyl-, Propionyl-, Pivaloyl-, (Methyl oder Aethyl)-carbamoyl-Derivate. Salze sind vorzugsweise Alkalimetallsalze, z. B. Natrium- oder Kaliumsalze von 1- oder 5-unsubstituierten Verbindungen ($R_1$ = H) und/oder von Carboxy-(phenyl oder phenylen)-Verbindungen, oder Säureadditionssalze von allen genannten Verbindungen mit Säuren, insbesondere mit den weiter unten genannten Säuren.

In den oben oder nachfolgend genannten $C_1$-$C_7$-Alkylresten sind solche mit höchstens 7, vorzugsweise 4, insbesondere 1 oder 2 Kohlenstoffatomen zu verstehen.

Die Verbindungen der Erfindung zeigen wertvolle pharmakologische Eigenschaften, nämlich psychoaktive Wirkungen, in erster Linie antidepressive oder anxiolytische Eigenschaften. Diese können

durch in vitro oder in vivo Testmethoden, vorzugsweise an Säugetieren, z. B. Mäusen, Ratten oder Affen, als Testobjekte, nachgewiesen werden. Die genannten Verbindungen können ihnen enteral oder parenteral, vorzugsweise oral, oder subkutan, intravenös oder intraperitoneal, z. B. durch Steckkapseln oder in Form von Stärke enthaltenden Suspensionen bzw. wässerigen Lösungen oder Suspensionen, verabreicht werden. Die verwendete Dosis kann in einem Bereich von zwischen 0,01 und 100 mg/kg/Tag, vorzugsweise 0,05 bis 5 mg/kg/Tag, insbesondere zwischen 0,1 und 0,5 mg/kg/Tag, liegen.

Die genannten antidepressiven Eigenschaften können an der Maus gemäss dem « Behavioral Despair »-Test (Arch. Int. Pharmacodyn. Ther. 229 (2), 327-336, Oktober 1977) nachgewiesen werden. In diesem Test wird das Versuchstier in einem engen Zylinder, aus welchem es nicht entkommen kann, zum Schwimmen gezwungen, wodurch ein depressiver Zustand ausgelöst wird. Nach einer kurzen Periode der lebhaften Aktivität nimmt die Maus eine charakteristische bewegungslose Körperstellung an, die leicht erkennbar ist. Das Eintreten dieser Immobilität wird durch die Verbindungen dieser Erfindung, durch andere tricyclische Antidepressiva, Monoaminoxydase hemmende Mittel, atypische Antidepressiva und durch den elektrokonvulsiven Schock vermindert.

Die anxiolytischen Effekte werden routinemässig, gemäss dem klassischen Metrazol-Antagonismus-Test an der Ratte, oder gemäss dem Cook-Davidson-Konflikt-Verfahren, in welchem man männliche Wistar-Ratten verwendet, festgestellt. Diese Ratten werden durch Diät, aber keine Einschränkung der Wasserzufuhr, bei 80 % des normalen Körpergewichtes gehalten. Sie werden trainiert, einen Hebel innerhalb einer Konditionierungskammer zu drücken. Die Kammer enthält auch einen Tropfer für Flüssigkeit, eine Lichtquelle, einen Lautsprecher und einen Gitterboden. Sowohl der Hebel als auch das Gitter sind an eine Elektroschock-Quelle angeschlossen. Die Kammer befindet sich in einem schallgedämpften Raum, in welchem im Laufe des Testverfahrens eine weisse Geräuschquelle die auswertigen Höreinflüsse abschirmt. Jede Testperiode von 47 Minuten besteht aus zwei alternierenden Programmen. Das erste ist ein Variables Intervall (VI)-Programm von 30 Sekunden, das sich während 5 Minuten wiederholt. In diesem Programm wird 30 Sekunden nach der ersten Hebelbetätigung eine gesüsste Kondensmilchration der Ratte abgegeben. Die vom Wirkstoff ausgelöste Abnahme dieser Leistung der Hebelbetätigung wird als Hinweis auf das neurologische Defizit betrachtet. Unmittelbar nach dem VI-Programm werden gleichzeitig ein Ton von 1 000 Hz und ein Lichtsignal aktiviert, welche den Anfang des zweiten, sogenannten Fixed Ratio (FR)-Programmes anzeigen, welches 2 Minuten dauert. In diesem Zeitintervall wird unmittelbar nach dem zehnten Hebeldruck, gleichzeitig mit der Milchration ein elektrischer Fuss-Schock verabreicht, wodurch eine Konfliktsituation entsteht. Die Intensität des genannten Schocks liegt zwischen 2,0 und 3,6 mA. Sie variiert für jedes Versuchstier, um es während der ganzen Sitzungsperiode auf ungefähr 25 bis 100 Hebeldrücke innerhalb dieses Programmes einzustellen. Die vom Wirkstoff hervorgerufene Steigerung dieser Leistung während des FR-Programms gilt als Hinweis auf die anxiolytische Wirkung, wie sich dies bei den Verbindungen der vorliegenden Erfindung zeigt.

Die anxiolytischen Effekte der neuen Verbindungen lassen sich auch im « Diazepam Receptor-Binding »-Versuch in vitro, z. B. wie in Nature 266, 732 (1977) oder in Proc. Nat. Acad. Sci. USA 74, 3805 (1975) beschrieben, beurteilen. Das Diazepam bindet sich spezifisch und mit hoher Affinität an rohe synaptosomale Membranpräparate aus dem Vorderhirn der Ratte. Diese Bindung wird durch andere anxiolytische Verbindungen, z. B. durch pharmakologisch aktive Benzodiazepine gehemmt. Verwendet man mit Tritium markiertes Diazepam, so lässt sich die Wechselwirkung von anderen Wirkstoffen mit dem genannten Rezeptor wie folgt leicht beurteilen : Membranen aus dem Vorderhirn der Ratte werden bei 0-5° mit Tritium markiertem Diazepam und mit verschiedenen Konzentrationen der getesteten Substanzen in einem physiologischen Medium, bei einem pH-Wert von 7,5, inkubiert. Die Membranen, welche die Rezeptoren mit verschiedenen Mengen des mit Tritium markierten Diazepams enthalten, werden auf Glasfaserfiltern filtriert. Diese werden dann in einem Flüssigkeits-Scintillation-Zähler geschüttelt. Die Konzentration der erfindungsgemässen Verbindungen, welche zur 50 %igen Hemmung der spezifischen Bindung von 2 nM mit Tritium markiertem Diazepam notwendig ist, d. h. die $IC_{50}$ (Inhibitory Concentration) lässt sich graphisch berechnen. Diese Konzentration geht bis zu einem Bereich von ungefähr 0,6 nM hinunter. Diese Konzentration ist in ihrer Grössenordnung niedriger als diejenige von Diazepam (5 nM) und fast vier Potenzen niedriger als der Wert für das Chlordiazepoxyd (400 nM).

Dementsprechend sind die Verbindungen der Erfindung wertvoll in der Behandlung von mentalen Depressionen und vorzugsweise für die Bekämpfung von Angstzuständen, die ähnlich denjenigen, welche mit Diazepam behandelt werden, sind. Im Gegensatz zu Diazepam besitzen die genannten Verbindungen der Erfindung bei Dosen, bei welchen die angstlösende Wirkung bereits zustandekommt, offensichtlich keine Tendenz ein neurologisches Defizit auszulösen. Die neuen Verbindungen können überdies als Zwischenprodukte zur Herstellung von anderen wertvollen, insbesondere von pharmakologisch wirksamen Präparaten, eingesetzt werden.

Bevorzugte Verbindungen sind diejenigen der Formeln I und II, worin $R_3$ Wasserstoff oder einen oder zwei Substituenten der Gruppe $C_1$-$C_7$-Alkyl, $C_1$-$C_7$-Alkoxy, $C_1$-$C_7$-Alkylthio, Hydroxy, Halogen, Trifluormethyl, Nitro, Amino, Mono- oder Di-$C_1$-$C_7$-alkylamino, Cyan, Carbamoyl oder Carboxy bedeutet, R für unsubstituiertes oder durch einen oder zwei der für $R_3$ angegebenen Reste substituiertes Phenyl, Pyridyl, $C_1$-$C_7$-Alkylpyridyl oder Halogenpyridyl steht, $R_1$ Wasserstoff, $C_1$-$C_7$-Alkyl oder (Hydroxy, di-$C_1$-$C_7$-Alkylamino oder $R_3$-Phenyl)-$C_1$-$C_7$-Alkyl bedeutet, worin die Hydroxy- oder Aminogruppe vom Ringstickstoffatom durch mindestens 2 Kohlenstoffatome getrennt ist, und $R_2$ für Wasserstoff oder $C_1$-$C_7$-Alkyl steht; $C_1$-$C_7$-Alkanoyl-, Carbamoyl-, Mono- oder Di-$C_1$-$C_7$-alkylcarbamoyl-Derivate der genannten (Hydroxy oder Amino)-(phenyl oder phenylen)-Verbindungen; oder die Salze, insbesondere pharmazeutisch verwendbaren Alkalimetall- oder Säureadditionssalze davon.

Bevorzugt sind weiter Verbindungen der Formel III

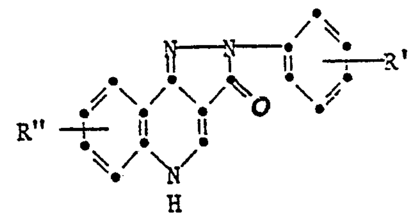

(III)

worin R″ Wasserstoff, Alkyl oder Alkoxy mit jeweils 4 Kohlenstoffatomen, Hydroxy, Fluor, Chlor, Brom oder Trifluormethyl bedeutet, R′ für Wasserstoff, Alkyl oder Alkoxy mit je 4 Kohlenstoffatomen, Hydroxy, Fluor, Chlor, Brom, Trifluormethyl, Nitro, Amino, Monoalkylamino oder Alkylcarbamoylamino mit je höchstens 4 Kohlenstoffatomen, oder Cyan steht; oder die Salze, insbesondere die pharmazeutisch verwendbaren Alkalimetall- oder Säureadditionssalze davon.

Hervorzuheben sind die Verbindungen der Formel III, worin R″ Wasserstoff oder 8-(Methyl, Methoxy, Fluor oder Chlor) bedeutet, und R′ für Wasserstoff steht oder 4-Fluor bedeutet, wenn R″ 8-Chlor ist; oder die Salze, insbesondere die pharmazeutisch verwendbaren Alkalimetall- oder Säureadditionssalze, hinsichtlich ihrer überwiegenden antidepressiven Aktivität.

Besonders hervorzuheben sind Verbindungen der Formel III, worin R″ Wasserstoff oder 8-(Methyl, Methoxy, Fluor oder Chlor) bedeutet, und R′ für 4-(Methyl, Methoxy, Chlor, Brom, Amino oder Cyan) steht oder 4-Fluor bedeutet, wenn R″ von 8-Chlor verschieden ist; oder die Salze, insbesondere pharmazeutisch verwendbaren Alkalimetall- oder Säureadditionssalze davon, hinsichtlich ihrer überwiegenden anxiolytischen Aktivität.

Bevorzugt sind weiter Verbindungen der allgemeinen Formel III, worin R′ Wasserstoff bedeutet, und R″ für Wasserstoff, 8-(Methoxy, Fluor oder Chlor) steht oder R′ 4-Fluor bedeutet und R″ 8-Chlor ist; oder die Salze, insbesondere ihr Natriumsalz, Hydrochlorid oder Methansulfonat, hinsichtlich ihrer überwiegenden antidepressiven Aktivität.

Besonders bevorzugt sind Verbindungen der Formel III, worin R′ 4-(Methyl, Chlor oder Amino) bedeutet und R″ für Wasserstoff steht; oder die Salze, insbesondere ihr Natriumsalz, Hydrochlorid oder Mesylat, hinsichtlich ihrer überwiegenden anxiolytischen Aktivität.

Die Verbindungen der Erfindung werden nach an sich bekannten Methoden, z. B. dadurch hergestellt, dass man

a) Verbindungen der Formel IV

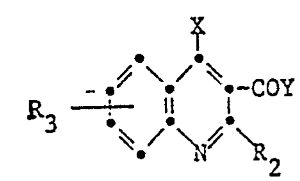

(IV)

worin X —NH—NH—R bedeutet und Y für Hydroxy oder $C_1$-$C_7$-Alkoxy steht; oder X Halogen bedeutet und Y für $H_2N$—N—R steht; oder X $C_1$-$C_7$-Alkoxyamino oder Azido bedeutet und Y für NH—R steht, ringschliesst, und, wenn erwünscht, eine erhaltene Verbindung oder ein Alkalimetallsalz davon, mit einem reaktionsfähigen Ester eines Alkohols $R_1$—OH umsetzt.

Der Ringschluss der genannten Säuren oder Amide IV wird durch Erhitzen auf Temperaturen zwischen 80 und 180°, vorzugsweise in Gegenwart von inerten Lösungsmitteln, z. B. aliphatischen oder aromatischen Kohlenwasserstoffen und/oder Aethern, wie Toluol, Xylole, Biphenyle und/oder Diphenyläther, wobei das gebildete Wasser oder Alkanol abdestilliert wird, durchgeführt. Die genannten Hydrazide IV werden in analoger Weise, jedoch vorzugsweise unter basischen Bedingungen, z. B. in Gegenwart von wässerigen Alkalimetallhydroxyden, um die entstandenen Halogenwasserstoffsäuren zu neutralisieren, ringgeschlossen. Der Ringschluss der genannten Amide IV wird durch Erhitzen auf Temperaturen zwischen 120 und 300°, vorzugsweise zwischen 200 und 250°, insbesondere auch in Gegenwart der genannten inerten Lösungsmittel, vorgenommen.

Einige der Ausgangsstoffe der Formel IV sind neu, sie können jedoch leicht aus den bekannten Vorstufen, worin X Hydroxy bedeutet, durch Kondensation mit entsprechenden Aryl-hydrazinen, hergestellt werden. Solche Methoden sind z. B. in den Beispielen illustriert oder in J. Med. Chem. 12, 1096 (1969) oder C.R. Acad. Sc. Paris, t. 280, C, 1385 (1975) beschrieben. Die genannten Hydrazide werden durch Kondensation von 4-Chlorchinolin-3-carbonsäurechloriden und β-acylierten Arylhydrazi-

nen, z. B. Trifluoracetaten, welche unter den Ringschlussbedingungen hydrolysieren, erhalten werden. Die genannten Amide werden vorzugsweise durch Kondensation von 4-Halogenchinolin-3-carbonsäurehalogeniden mit R-Aminen und nachfolgend mit O-$C_1$-$C_7$-Alkyl-hydroxylaminen oder Alkalimetallaziden hergestellt.

Ein weiteres Verfahren zur Herstellung der erfindungsgemässen Verbindungen besteht darin, dass man

b) Verbindungen der Formel V

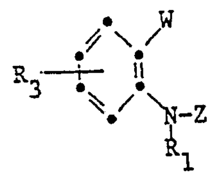

(V)

worin die beiden Symbole W und $R_1$ Wasserstoff bedeuten und Z für die Gruppierung

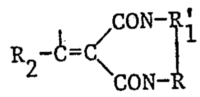

steht, worin $R_1'$ $C_1$-$C_7$-Alkyl bedeutet ; oder W die Gruppierung

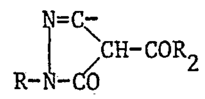

ist und Z für Wasserstoff steht ; oder W die Gruppierung

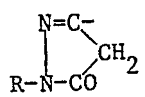

bedeutet und Z für $R_2$-CO oder $R_1$-N—Z zusammengenommen für Isocyano steht, kondensiert, und, wenn erwünscht, eine erhaltene Verbindung in eine andere Verbindung der Erfindung umwandelt, und/oder, wenn erwünscht, eine erhaltene Verbindung in ihr Salz oder ein erhaltenes Salz in die freie Verbindung oder in ein anderes Salz überführt, und/oder, wenn erwünscht, ein erhaltenes Gemisch von Isomeren in die einzelnen Isomeren auftrennt.

Die Ringschluss-Kondensation von Ausgangsstoffen, in welchen W Wasserstoff bedeutet, wird vorzugsweise mit starken aprotischen Kondensationsmitteln, z. B. Polyphosphorsäureniederalkylestern, durchgeführt. Verwendet man Ausgangsstoffe, in welchen Z Wasserstoff bedeutet, so wird das bei dem Ringschluss entstandene Wasser vorzugsweise azeotropisch entfernt. Man arbeitet vorzugsweise in den oben genannten Kohlenwasserstoffen und/oder Aethern, wenn erwünscht, in Gegenwart von konventionellen Molekularsieben, und/oder von katalytischen Mengen einer Säure, z. B. Chlorwasserstoffsäure.

Schliesslich werden Verbindungen der Formel V, in welchen W und Z Kohlenstoff enthält, unter neutralen Bedingungen, gegebenenfalls in Gegenwart von Dehydratisierungsmitteln, z. B. Thionylhalogeniden, Phosphoroxyhalogeniden oder Polyphosphorsäure-niederalkylestern, ringgeschlossen.

Der Ausgangsstoff der Formel V ist auch neu, er kann jedoch nach an sich bekannten Methoden, z. B. durch Kondensation von 1-Arylpyrazolidin-3,5-dionen mit Orthoameisensäure-äthylester und einem Anilin, hergestellt werden. Der genannte zweite Ausgangsstoff der Formel V kann analog zu dem in Khim. Geterotsikl. Spedin. Akad. Nauk. Latv. SSR. 1965, 587, jedoch durch Verwendung von analogen Verbindungen mit einer o-Nitrogruppe, welche nachfolgend mit katalytisch aktiviertem Wasserstoff reduziert wird, erhalten werden. Die letzte Gruppe von Ausgangsstoffen der Formel V wird ähnlich, ausgehend von genannten üblichen 1-R-3-(o-Nitrophenyl)-5-pyrazolonen, durch Reduktion, N-Acylierung und, wenn erwünscht, ihre Dehydratisierung zu den genannten Isonitrilen mit Phosgen oder Dimethylformamid/Thionylhalogenid-Gemischen, hergestellt.

Die erhaltenen Verbindungen der Erfindung können in an sich bekannter Weise ineinander übergeführt werden. So können z. B. Verbindungen, in welchen $R_1$ Wasserstoff bedeutet, in 1-Stellung mit einem reaktionsfähigen Ester von $R_1$—OH substituiert werden. Diese Ester sind z. B. von Halogenwasserstoff-, aliphatischen oder aromatischen Sulfonsäuren abgeleitet, und bedeuten z. B. $R_1$-(Halogenide, Sulfate, aliphatische oder aromatische Sulfonate), wie Methyljodid, Dimethylsulfat, Benzylchlorid oder Methansulfonsäure- oder Toluolsulfonsäuremethylester. Man erhält dabei 1-substituierte Verbindungen der Formel I. Solche der Formel II werden ähnlich hergestellt aus entsprechenden Alkalimetallsalzem, z. B. in welchen $R_1$ Natrium oder Kalium bedeutet, wobei dei Substitution in 5-Stellung stattfindet. Weiter können erhaltene $C_1$-$C_7$-Alkoxy-Verbindungen zu den entsprechenden

Phenolen mit starken Halogenwasserstoffsäuren, z. B. Bromwasserstoffsäure, hydrolysiert werden. Erhaltene Nitro-Verbindungen können zu den entsprechenden Aminen mit katalytisch aktiviertem oder nascierendem Wasserstoff, z. B. Wasserstoff in Gegenwart von Edelmetall-Katalysatoren, z. B. Nickel-, Palladium- oder Platinkatalysatoren oder mit Wasserstoff, der durch Einwirkung von reaktionsfähigen Metallen auf Alkohole oder Säuren erzeugt wird, z. B. Zink und Halogenwasserstoffsäuren, reduziert werden. Die genannten Amine können gleich wie für die Verbindungen mit $R_1$ = H beschrieben, oder durch reduktive Alkylierung, alkyliert, oder z. B. mit entsprechenden, reaktionsfähigen Säurederivaten, z. B. Anhydriden, Halogeniden oder Isocyanaten, acyliert werden. Erhaltene Nitrile können in an sich bekannter Weise, z. B. durch Behandlung mit wässerigen Alkalimetallhydroxydlösungen, z. B. wässeriger Natriumhydroxydlösung, Aethanol und Wasserstoffsuperoxyd, in die entsprechenden Amide übergeführt, oder durch Hydrolyse, z. B. mit wässeriger Alkalimetallhydroxydlösungen, wie wässeriger Natriumhydroxydlösung, in die entsprechenden Säuren umgewandelt werden.

Schliesslich kann eine erhaltene Verbindung entweder in ihre Alkalimetallsalze, vorzugsweise unter Verwendung von Alkali-metall-hydriden, -hydroxyden oder -$C_1$-$C_7$-Alkoxyden, oder in ihre Säureadditionssalze (insbesondere im Falle von Amino-substituierten Verbindungen), vorzugsweise unter Verwendung von anorganischen oder organischen Säuren, welche therapeutisch verwendbare Salze ergeben, übergeführt werden. Solche Säuren sind z. B. starke anorganische Säuren, wie Halogenwasserstoffsäuren, z. B. Chlorwasserstoff- oder Bromwasserstoffsäure, oder Schwefel-, Phosphor-, Salpeter- oder Perchlorsäure ; oder aliphatische oder aromatische Carbon- und Sulfonsäuren, z. B. Ameisen-, Essig-, Propion, Bernstein-, Glykol-, Milch-, Aepfel-, Wein-, Zitronen-, Malein-, Hydroxymalein-, Brenztrauben-, Phenylessig-, Benzoe-, 4-Aminobenzoe-, Anthranil-, 4-Hydroxybenzoe-, Salicyl-, 4-Aminosalicyl-, Pamoe-, Nikotin-, Methansulfon-, Aethansulfon-, Hydroxyäthansulfon-, Aethylensulfon-, Halogenbenzolsufon-, Toluolsulfon-, Naphthalinsulfon-, Sulfanil-, Cyclohexylsulfaminsäure ; oder die Ascorbinsäure. Diese oder andere Salze, z. B. Pikrate, können auch in der Reinigung von Amino-Basen verwendet werden. Die Basen werden in ihre Salze übergeführt, die Salze abgetrennt und die freien Verbindungen aus den Salzen freigesetzt. Infolge der engen Beziehungen zwischen den neuen Verbindungen in freier Form und in Form ihrer Salze sind im vorausgegangenen und nachfolgend unter freien Verbindungen und Salzen sinn- und zweckgemäss gegebenenfalls auch die entsprechenden Salze bzw. freien Verbindungen zu verstehen. Säureadditionssalze von Verbindungen, welche keine basischen Substituenten, z. B. keine Aminogruppe, aufweisen, hydrolysieren in der Regel in ungefähr neutralen wässerigen Medien.

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, Kondensationsmitteln oder Neutralisationsmitteln, und/oder in einer inerten Atmosphäre, unter Kühlung, bei Zimmertemperatur oder bei erhöhten Temperaturen, vorzugsweise bei dem Siedepunkt des verwendeten Lösungsmittels, bei normalem oder erhöhtem Druck durchgeführt.

Erhaltene Isomerengemische können nach an sich bekannten Methoden, z. B. durch fraktionierte Destillation, Kristallisation und/oder Chromatographie, in die einzelnen Isomeren getrennt werden.

Die Erfindung betrifft ebenfalls Abänderungen des vorliegenden Verfahrens, wonach ein auf irgendeiner Stufe des Verfahrens erhältliches Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, z. B. solche der Formel IV mit X = NH—NH—R, ausgehend von ihren Vorstufen, in welchen X Chlor bedeutet, oder worin ein Ausgangsstoff in Form eines Salzes verwendet wird.

Beim Verfahren der vorliegenden Erfindung werden vorzugsweise solche Ausgangsstoffe verwendet, welche zu den eingangs als besonders wertvoll geschilderten Verbindungen, insbesondere solchen der Formel III führen.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z. B. Laktose, Dextrose, Rohrzucker, Mannitol, Sorbitol, Cellulose und/oder Glycin, und Schiermitteln, z. B. Kieselerde, Talk, Stearinsäure oder Salze davon, wie Magnesium- oder Calciumstearat, und/oder Polyäthylenglykol, aufweisen ; Tabletten enthalten ebenfalls Bindemittel, z. B. Magnesiumaluminiumsilikat, Stärke-Paste, Gelatine, Traganth, Methylcellulose, Natriumcarboxymethylcellulose und/oder Polyvinylpyrrolidon, und, wenn erwünscht, Sprengmittel, z. B. Stärken, Agar, Alginsäure oder ein Salz davon, Enzyme der Bindemittel und/oder Brausemischungen, oder Adsorptionsmittel, Farbstoffe, Geschmackstoffe und Süssmittel. Injizierbare Präparate sind vorzugsweise isotonische wässerige Lösungen oder Suspensionen, und Suppositorien in erster Linie Fettemulsionen oder -suspensionen. Die pharmakologischen Präparate können sterilisiert sein und/oder Hilfsstoffe, z. B. Konservier-, Stabilisier-, Netz- und/oder Emulgiermittel, Löslichkeitsvermittler, Salze zur Regulierung des osmotischen Druckes und/oder Puffer enthalten. Die vorliegenden pharmazeutischen Präparate, die, wenn erwünscht, weitere pharmakologisch wertvolle Stoffe enthalten können, werden in an sich bekannter Weise, z. B. mittels konventioneller Misch-, Granulier- oder

Dragierverfahren, hergestellt und enthalten von etwa 0,1 bis etwa 75 %, insbesondere von etwa 1 bis 50 %, des Aktivstoffes.

Die folgenden Beispiele dienen zur Illustration der Erfindung. Temperaturen werden in Celsiusgraden angegeben und die Angaben über Teile betreffen Gewichtsteile. Wenn nicht anders definiert, wird das Eindampfen von Lösungsmitteln unter vermindertem Druck, z. B. zwischen 0,1 und 15 mm Hg (0,133 und 20 mbar), durchgeführt.

Die in den folgenden Beispielen genannten Verbindungen, welche einen zu hohen Schmelzpunkt aufweisen, sind durch ihre I.R.- oder NMR-Spektren charakterisiert.

## Beispiel 1

Ein Gemisch von 1 681 g 4-Chlorchinolin-3-carbonsäureäthylester, 1 017 g p-Chlorphenyl-hydrazin und 25 000 ml Xylol wird unter Rühren in einer Stickstoffatmosphäre 24 Stunden auf 105° erhitzt. Die erhaltene Suspension wird auf 20° gekühlt, mit 14 000 ml 2-normaler wässeriger Natriumhydroxidlösung versetzt, 15 Minuten gerührt und mit 30 000 ml Wasser verdünnt. Das Rühren wird 1 Stunde fortgesetzt, die wässerige Phase abgetrennt, fünfmal mit je 8 000 ml Diäthyläther gewaschen, filtriert und das Filtrat mit einer Lösung von 1 600 g Ammoniumchlorid in 8 000 ml Wasser, unter Rühren in einer Stickstoffatmosphäre behandelt. Die erhaltene Suspension wird über Nacht bei Zimmertemperatur gerührt, filtriert und der Rückstand fünfmal mit 12 000 ml heissem Wasser gewaschen. Der Rückstand wird bei 5 mm Hg und 90° getrocknet und 1 665 g davon in 8 400 ml Dimethylformamid bei 130° gelöst. Die Lösung wird filtriert und unter Rühren auf Zimmertemperatur abkühlen gelassen. Die erhaltene Suspension wird filtriert, zweimal mit je 500 ml kaltem Dimethylformamid und viermal mit 1 000 ml Diäthyläther gewaschen und der Rückstand bei 0,1 mm Hg (0,133 mbar) und 100° getrocknet. Man erhält das 2-(p-Chlorphenyl)-pyrazolo [4,3-c]-chinolin-3(5H)-on der Formel

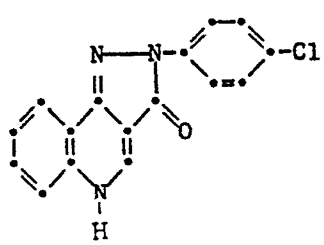

welches unter Zersetzung bei 324-327° schmilzt.

Der Ausgangsstoff wird wie folgt (gemäss einer allgemeinen anwendbaren Methode) hergestellt: Man gibt zu 1 272 g Anilin innerhalb 20 Minuten unter Rühren, 2 953 g Aethoxymethylen-malonsäurediäthylester und setzt das Rühren 135 Minuten bei 90-92° fort. Das entstandene Aethanol wird 4 Stunden bei 10 mm Hg 13,33 mbar und 80° abdestilliert. Das als Rückstand erhaltene Oel laßtman auf Platten kristallisieren. Die Kristalle werden pulverisiert und bei 5 mm Hg (6,67 mbar und Zimmertemperatur getrocknet. Man erhält den Phenylaminomethylen-malonsäure-diäthylester, der bei 45-46° schmilzt.

Man gibt 1 085 g der letztgenannten Verbindung innerhalb 45 Minuten zu 10 850 ml eines eutektischen Gemisches von Diphenyläther-Biphenyl (73,5 : 26,5 Gewichtsteile) unter Stickstoff, unter Rühren bei 215-220°. Nach der abgeschlossenen Hinzufügung wird die Temperatur auf 238° erhöht und das entstandene Gemisch von Aethanol und Diphenyläther innerhalb 4 Stunden in einem Abscheider aufgefangen (ungefähr 390 ml). Man lässt das Reaktionsgemisch unter Rühren auf Zimmertemperatur abkühlen. Die erhaltene Suspension wird filtriert, der Rückstand zweimal mit je 500 ml Diäthyläther gewaschen und bei 0,1 mm Hg (0,133 mbar) und 85° getrocknet. Man erhält den 4-Hydroxychinolin-3-carbonsäure-äthylester, der bei 276-280° schmilzt.

Man gibt 1 630 g der letztgenannten Verbindung zu 2 463 ml Phosphoroxychlorid innerhalb 30 Minuten, unter Rühren in einer Stickstoffatmosphäre. Das Gemisch wird 15 Minuten bei 70° und 2 Stunden bei 95° gerührt und dann die Flüssigkeit bei 11 mm Hg (14,7 mbar) und 60° abdestilliert. Der Rückstand wird in 8 000 ml Methylenchlorid gelöst, die Lösung auf 0° gekühlt und mit 5 000 g zerdrücktem Eis behandelt. Das Gemisch wird gerührt, mit 3 000 ml 50 %iger wässeriger Natriumhydroxydlösung unter 15° behandelt, und wenn der pH-Wert 12 erreicht ist, die organische Schicht abgetrennt. Diese wird zweimal mit je 2 000 ml Wasser, einmal mit 2 000 ml gesättigter wässeriger Natriumchloridlösung gewaschen, getrocknet und eingedampft. Man lässt das als Rückstand erhaltene Oel auf Platten kristallisieren. Diese werden pulverisiert und bei 0,1 mm Hg (0,133 mbar bei Zimmertemperatur getrocknet. Man erhält den 4-Chlorchinolin-3-carbonsäure-äthylester, der bei 44-46° schmilzt.

Eine Lösung von 1 445 g p-Chloranilin in 3 375 ml 38%iger Chlorwasserstoffsäure und 5 650 ml Wasser wird bei − 5° bis − 8° innerhalb einer Stunde, unter Rühren in einer Stickstoffatmosphäre, mit einer Lösung von 793 g Natriumnitrit in 3 300 ml Wasser versetzt. Nach 15 Minuten gibt man 7 617 g Stannochlorid in 9 000 ml 38 %iger Chlorwaseerstoffsäure innerhalb 30 Minuten und unter 25° dazu. Die erhaltene Suspension wird eine Stunde in einem Eisbad gerührt und filtriert. Der Rückstand wird in 30 000 ml Wasser suspendiert und unter Rühren in einer Stickstoffatmosphäre mit 5 000 g festem

Natriumhydroxyd innerhalb einer Stunde bei 0-25° versetzt. Das Gemisch wird zweimal mit 8 000 ml Diäthyläther extrahiert, die vereinigten Extrakte werden zweimal mit 4 000 ml Wasser und einmal mit gesättigter wässeriger Natriumchloridlösung gewaschen, getrocknet, filtriert und eingedampft. Der Rückstand wird bei 5 mm Hg (6,67 mbar) und Zimmertemperatur getrocknet. Man erhält das p-Chlorphenyl-hydrazin, welches bei 82-87° schmilzt.

Beispiel 2

Ein Gemisch von 1 g 2-(p-Chlorphenyl)-pyrazolo [4,3-c] chinolin-3(5H)-on und 3,38 ml 1-normaler wässeriger Natriumhydroxydlösung wird in einer Stickstoffatmosphäre über Nacht bei Zimmertemperatur gerührt. Die erhaltene Lösung wird filtriert, eingedampft und der Rückstand unter vermindertem Druck getrocknet. Man erhält das entsprechende Natriumsalz, welches bei 280-284° schmilzt.

Beispiel 3

Ein Gemisch von 1 g 2-(p-Chlorphenyl)-pyrazolo [4,3-c] chinolin-2(5H)-on, 20 ml Trifluoressigsäure und 0,325 g Methansulfonsäure wird eine Stunde bei Zimmertemperatur gerührt und eingedampft. Der Rückstand wird mit Diäthyläther trituriert und abfiltriert. Man erhält das entsprechende Methansulfonat, welches bei 250-255° schmilzt.

Beispiel 4

Ein Gemisch von 3 g 2-(p-Chlorphenyl)-pyrazolo [4,3-c]-chinolin-3(5H)-on und 100 ml Dimethylsulfat wird bei 110-130° 2 Stunden gerührt und eingedampft. Der Rückstand wird in 1-normaler wässeriger Natriumhydroxydlösung gelöst, die Lösung mit Methylenchlorid extrahiert und der Extrakt eingedampft. Der Rückstand wird aus Diäthyläther umkristallisiert. Man erhält das 1-Methyl-2-(p-chlorphenyl)-pyrazolo [4,3-c] chinolin-3-on, welches bei 158-161° schmilzt.

Beispiel 5

Ein Gemisch von 5 g 2-(p-Chlorphenyl)-pyrazolo [4,3-c] chinolin-3(5H)-on, 0,81 g 50%igem Natriumhydrid in Mineralöl und 100 ml wasserfreiem Tetrahydrofuran wird 2 Stunden unter Rückfluss gekocht. Das Gemisch wird auf Zimmertemperatur gekühlt, unter Rühren mit 3 g Methyljodid versetzt und nach einer Stunde mit weiterem 1 g Methyljodid verrührt. Das Gemisch wird über Nacht bei Zimmertemperatur gerührt, filtriert und der Rückstand aus Tetrahydrofuran-Heptan umkristallisiert. Man erhält das 5-Methyl-2-(p-chlorphenyl)-pyrazolo-[4,3-c] chinolin-3-on, welches bei 322-323° schmilzt.

Beispiel 6

Ein Gemisch von 10 g 2-(p-Chlorphenyl)-pyrazolo [3,4-c] chinolin-3(5H)-on, 1,8 g 50%igem Natriumhydrid in Mineralöl und 250 ml 1,2-Dimethoxyäthan wird bis zur Auflösung bei 100° gerührt. Das Gemisch wird auf Zimmertemperatur gekühlt und mit 15 g 3-Dimethyl-amino-propylchlorid in 10 ml 1,2-Dimethoxyäthan versetzt. Das Gemisch wird bei 150° über Nacht gerührt, abgekühlt, die obenstehende Lösung dekantiert und der Rückstand mit der genannten Lösung behandelt. Man erhält das 5-(3-Dimethylaminopropyl)-2-(p-chlorphenyl)-pyrazolo-[4,3-c] chinolin-3-on, welches bei 180-191° schmilzt.

In analoger Weise erhält man das 5-(2-Dimethylaminoäthyl)-2-(p-chlorphenyl)-pyrazolo [4,3-c] chinolin-3-on, welches bei 184-186° schmilzt.

Beispiel 7

Ein Gemisch von 2 g 2-(p-Chlorphenyl)-pyrazolo [4,3-c] chinolin-3(5H)-on, 0,33 g 50%igem Natriumhydrid in Mineralöl und 50 ml 1,2-Dimethoxyäthan wird bei 100° bis zur Auflösung gerührt. Das Gemisch wird auf Zimmertemperatur gekühlt, mit 3,9 g o-Fluorbenzylchlorid in 2 ml 1,2-Dimethoxyäthan versetzt und das Gemisch 4 Stunden unter Rückfluss gekocht. Das Gemisch wird auf Zimmertemperatur gekühlt, filtriert, der Rückstand mit Diäthyläther gewaschen, in 10 ml 1-normaler wässeriger Natriumhydroxydlösung aufgeschlämmt, wieder filtriert, mit Wasser gewachen und getrocknet. Man erhält das 5-(o-Fluorbenzyl)-2-(p-chlorphenyl)-pyrazolo [3,4-c] chinolin-3-on, welches bei 338-339° schmilzt.

Beispiel 8

Ein Gemisch von 3,5 g 4-(2,4-Dichlorphenyl-hydrazino)-chinolin-3-carbonsäure-äthylester und 40 ml eutektischem Diphenyläther-Biphenyl wird 4 Stunden auf 175° erhitzt, auf Zimmertemperatur gekühlt und mit Diäthyläther verdünnt. Die erhaltene Suspension wird filtriert, der Rückstand mit Diäthyläther gewaschen und in 1-normaler wässeriger Natriumhydroxidlösung gelöst. Die Lösung wird mit Diäthyläther gewaschen, ihr pH-Wert mit Ammoniumchlorid auf 8,5 eingestellt und der erhaltene Niederschlag abgetrennt. Der Niederschlag wird nacheinander mit Wasser, Methanol und Diäthyläther gewaschen. Man erhält das 2-(2,4-Dichlorphenyl)-pyrazolo [4,3-c] chinolin-3(5H)-on, welches im IR-Spektrum Banden bei 890, 867, 845, 832, 816, 796, 775, 767, 756, 730 und 701 $cm^{-1}$ zeigt.

Der Ausgangsstoff wird wie folgt hergestellt : Ein Gemisch von 2,8 g 4-Chlorchinolin-3-carbonsäure-äthylester, 2,1 g 2,4-Dichlorphenyl-hydrazin und 40 ml eutektischem Diphenyläther-Biphenyl wird unter Rühren über Nacht auf 80-90° erhitzt. Das Gemisch wird auf Zimmertemperatur gekühlt, mit Diäthyläther verdünnt und der Niederschlag abgetrennt. Dieser wird in 1-normaler wässeriger Natriumhydroxydlösung aufgenommen, die Lösung mit Diäthyläther extrahiert, der Extrakt mit Wasser gewaschen, getrocknet, filtriert, konzentriert und der Niederschlag abgetrennt. Man erhält den 4-(2,4-Dichlorphenylhydrazino)-chinolin-3-carbonsäure-äthylester, der bei 151-153° schmilzt.

Beispiel 9

Ein Gemisch von 3,6 g 4-Chlor-2-methyl-chinolin-3-carbonsäureäthylester, 1,8 g Phenylhydrazin und 40 ml Xylol wird 4 Stunden unter Rückfluss gekocht, auf Zimmertemperatur gekühlt, mit Diäthyläther verdünnt und filtriert. Der Rückstand wird in 50 ml 2-normaler wässeriger Natriumhydroxydlösung gelöst, die Lösung mit Diäthyläther gewaschen und ihr pH-Wert mit Ammoniumchlorid auf 8,5 eingestellt. Der erhaltene Niederschlag wird abgetrennt und nacheinander mit heissem Wasser, Methanol und Diäthyläther gewaschen. Man erhält das 4-Methyl-2-phenyl-pyrazolo [4,3-c] chinolin-3(5H)-on, welches im IR-Spektrum Banden bei 874, 867, 858, 850, 822, 780, 765, 756, 750, 740 und 722 $cm^{-1}$ zeigt.

In analoger Weise wird auch das 4-Methyl-2-(p-chlorphenyl)-pyrazolo [4,3-c] chinolin-3(5H)-on hergestellt. F. 349-350°.

Beispiel 10

Ein Gemisch von 2,7 g 4-Chlor-6-methoxy-chinolin-3-carbonsäureäthylester, 1,4 g p-Fluorphenylhydrazin und 20 ml eutektischem Diphenyläther-Biphenyl wird 4 Stunden bei 160-165° erhitzt, dann auf Zimmertemperatur gekühlt und mit Diäthyläther verdünnt. Das als Niederschlag erhaltene kristalline Produkt wird abgetrennt, mit Diäthyläther sorgfältig gewaschen und getrocknet. Man erhält das 2-(p-Fluorphenyl)-8-methoxy-pyrazolo [4,3-c] chinolin-3(5H)-on-hydrochlorid, welches bei 322-324° schmilzt.

Beispiel 11

Ein Gemisch von 4 g 4-Chlorchinolin-3-carbonsäure-äthylester, 2,04 g 2-Hydrazino-pyridin und 50 ml eutektischem Diphenyläther-Biphenyl wird in einer Stickstoffatmosphäre 3 Stunden bei 110-130° gerührt. Das Gemisch wird auf Zimmertemperatur gekühlt, mit Diäthyläther verdünnt, filtriert, das feste Material mit Diäthyläther gewaschen und in 100 ml wässeriger Natriumhydroxydlösung gelöst. Die Lösung wird mit Diäthyläther gewaschen und ihr pH-Wert mit Ammoniumchlorid auf 8,5 eingestellt. Der erhaltene Niederschlag wird abgetrennt und nacheinander mit Wasser, Methanol und Diäthyläther gewaschen. Man erhält das 2-(2-Pyridyl)-pyrazolo [4,3-c] chinolin-3(5H)-on, welches im IR-Spektrum Banden bei 887, 865, 853, 788, 780, 765, 756, 737 und 726 $cm^{-1}$ zeigt.

In analoger Weise wird auch das 8-Fluor-2-(2-pyridyl)-pyrazolo [4,3-c] chinolin-3(5H)-on hergestellt, welches im IR-Spektrum Banden bei 895, 868, 826, 792, 776, 758 und 725 $cm^{-1}$ zeigt.

Der Ausgangsstoff für die letztgenannte Verbindung wird wie folgt hergestellt : Ein Gemisch von 28,9 g 6-Fluor-4-hydroxy-chinolin-3-carbonsäure-äthylester [J.A.C.S. 69, 371 (1947)] und 240 ml Phosphoroxychlorid wird 3 Stunden in einer Stickstoffatmosphäre unter Rückfluss gekocht. Es wird auf Zimmertemperatur gekühlt, die Lösung eingedampft und der Rückstand mit Eiswasser und Chloroform behandelt. Die organische Schicht wird getrocknet und eingedampft. Der Rückstand wird in wässeriger Natriumhydrogencarbonatlösung und Diäthyläther aufgenommen, die ätherische Schicht getrocknet und eingedampft. Man erhält den 4-Chlor-6-fluor-chinolin-3-carbonsäure-äthylester, der bei 55-57° schmilzt.

Beispiel 12

Ein Gemisch von 3 g 2-(p-Methoxyphenyl)-pyrazolo [4,3-c]-chinolin-3(5H)-on und 260 ml 48 %iger Bromwasserstoffsäure wird eine Stunde unter Rückfluss gekocht und auf ungefähr 50 ml konzentriert. Das Konzentrat wird auf Zimmertemperatur gekühlt, der Niederschlag abgetrennt, mit Methanol und Diäthyläther gewaschen und in verdünnter wässeriger Natriumhydroxydlösung aufgelöst. Die Lösung wird mit Diäthyläther gewaschen, dann ihr pH-Wert mit Ammoniumchlorid auf 8,5 eingestellt. Der erhaltene Niederschlag wird abgetrennt, mit Methanol, dann mit Diäthyläther gewaschen und getrocknet. Man erhält das 2-(p-Hydroxyphenyl)-pyrazolo [4,3-c] chinolin-3(5H)-on, welches bei 294-296° schmilzt.

Beispiel 13

Eine Lösung von 1,9 g 2-(p-Nitrophenyl)-pyrazolo [4,3-c]-chinolin-3(5H)-on in einem Gemisch von

18,6 ml 2-normaler wässeriger Natriumhydroxydlösung und 75 ml Aethanol wird über 0,2 g Platinoxyd bei 2,7 Atmosphären 6 Stunden hydriert. Das Gemisch wird filtriert, das Filtrat eingedampft, der Rückstand in Wasser aufgenommen und die Lösung mit Diäthyläther gewaschen. Ihr pH-Wert wird mit Ammoniumchlorid auf 8,5 eingestellt, der Niederschlag abgetrennt und nacheinander mit Methanol und Diäthyläther gewaschen und getrocknet. Man erhält das 2-(p-Aminophenyl)-pyrazolo [4,3-c] chinolin-3(5H)-on, welches IR-Banden bei 896, 885, 865, 840, 820, 815, 782, 776, 761, 740, 736 und 720 $cm^{-1}$ zeigt.

Beispiel 14

Ein Gemisch von 1,4 g 2-(p-Aminophenyl)-pyrazolo [4,3-c]-chinolin-3(5H)-on, 4,1 ml 37 %igem wässerigem Formaldehyd, 1 g Natrium-cyanborhydrid und 20 ml Acetonitril wird unter Rühren mit 0,6 g Eisessig versetzt. Das Gemisch wird bei Zimmertemperatur über Nacht weiter gerührt und dann mit Wasser verdünnt. Der erhaltene Niederschlag wird in verdünnter Natriumhydroxydlösung gelöst, die wässerige Lösung mit Diäthylather gewaschen und ihr pH-Wert mit wässeriger Ammoniumchloridlösung auf 8,5 eingestellt. Der erhaltene Niederschlag wird abgetrennt, zuerst mit Methanol, dann mit Diäthyläther gewaschen und getrocknet. Man erhält das 2-(p-Methylaminophenyl)-pyrazolo [4,3-c] chinolin-3(5H)-on, welches bei 302-304° schmilzt.

Beispiel 15

Ein Gemisch von 0,7 g 2-(p-Aminophenyl)-pyrazolo [4,3-c]-chinolin-3(5H)-on, 1,4 g Methylisocyanat und 25 ml Methanol wird 7 Stunden unter Rückfluss gekocht und über Nacht bei Zimmertemperatur stehen gelassen. Das Gemisch wird eingedampft, der Rückstand mit verdünnter wässeriger Natriumhydroxydlösung und Diäthyläther behandelt, die wässerige Lösung abgetrennt, mit Diäthyläther gewaschen und ihr pH-Wert mit Ammoniumchlorid auf 8,5 eingestellt. Der erhaltene Niederschlag wird abgetrennt, zuerst mit Methanol und dann mit Diäthyläther gewaschen und getrocknet. Man erhält das 2-(p-Methylcarbamoylaminophenyl)-pyrazolo [4,3-c] chinolin-3(5H)-on, welches IR-Banden bei 895, 869, 850, 825, 816, 812, 785, 780, 767 und 738 $cm^{-1}$ zeigt.

Beispiel 16

Man erhitzt 3 Stunden unter Rühren bei 115-120° ein Gemisch von 38,5 ml einer 0,17-molaren Lösung von 4-Chlor-chinolin-3-carbonsäure-äthylester in Xylol und 0,96 g p-Cyan-phenylhydrazin in 30 ml Xylol. Das Gemisch wird auf Zimmertemperatur gekühlt und mit 20 ml 1-normaler wässeriger Natriumhydroxydlösung und einer zur Auflösung des festen Materials genügenden Wassermenge gerührt. Die wässerige Schicht wird abgetrennt, zweimal mit Diäthyläther gewaschen und dann mit einer wässerigen Lösung von 1,07 g Ammoniumchlorid behandelt. Der erhaltene Niederschlag wird abgetrennt, mit Wasser gewaschen und getrocknet. Man erhält das 2-(p-Cyanphenyl)-pyrazolo-[4,3-c] chinolin-3(5H)-on, welches im IR-Spektrum Banden bei 885, 830, 780, 755 und 730 $cm^{-1}$ zeigt.

Beispiel 17

Ein Gramm 2-(p-Cyanphenyl)-pyrazolo [4,3-c] chinolin-3(5H)-on, 3,50 ml 1-normaler wässeriger Natriumhydroxydlösung und 10 ml Aethanol werden vermischt und bei Zimmertemperatur bis zur Auflösung des festen Materials gerührt. Die Lösung wird dann mit 1,4 ml 30 %igem Wasserstoffsuperoxyd behandelt, wobei sich gleich ein Niederschlag abscheidet. Das Gemisch wird 2 Stunden bei Zimmertemperatur gerührt, filtriert und das feste Material aus Dimethylformamid umkristallisiert. Man erhält das 2-(p-Carbamoylphenyl)-pyrazolo [4,3-c] chinolin-3(5H)-on, welches im IR-Spektrum Banden bei 885, 853, 830, 785, 775, 752 und 732 $cm^{-1}$ zeigt.

Beispiel 18

Ein Gramm 2-(p-Cyanphenyl)-pyrazolo [4,3-c] chinolin-3(5H)-on und 50 ml 2-normaler wässeriger Natriumhydroxydlösung werden vermischt und 3 Stunden unter Rückfluss gekühlt. Die Lösung wird mit 50 ml Chlorwasserstoffsäure angesäuert, filtriert und der abgetrennte Niederschlag getrocknet. Dieser wird in 25 ml 1-normaler wässeriger Natriumhydroxydlösung aufgenommen und die Lösung mit 1-normaler Chlorwasserstoffsäure auf den pH-Wert 6-7 eingestellt. Das erhaltene feste Material wird abfiltriert, mit Wasser trituriert und getrocknet. Man erhält das 2-(p-Carboxyphenyl)-pyrazolo [4,3-c] chinolin-3(5H)-on 3/2 hydrat, welches im IR-Spektrum Banden bei 886, 858, 820, 780, 770, 760 und 730 $cm^{-1}$ zeigt.

Beispiel 19

Der pH-Wert einer Lösung von 1 g 4-Chlor-chinolin-3-(N-phenyl-N-trifluoracetamido)-carboxamid in einer minimalen Menge 50 %igem wässerigem Tetrahydrofuran wird durch Zugabe von Lithiumhydroxyd

auf 10 eingestellt. Das Gemisch wird 48 Stunden bei Zimmertemperatur gerührt, zwecks Beseitigung des grössten Teils des Tetrahydrofurans konzentriert, mit Dichlormethan gewaschen und durch Ansäuern mit verdünnter Chlorwasserstoffsäure auf den pH-Wert 3 eingestellt. Der erhaltene Niederschlag wird abgenutscht und durch präparative Dünnschichtchromatograhie auf Silicagel gereinigt. Man eluiert mit Toluol : Aethanol : konz. Ammoniumhydroxyd (70 : 30 : 3). Man erhält das 2-Phenyl-pyrazolo [4,3-c] chinolin-3(5H)-on, welches bei 326-328° schmilzt.

Der Ausgangsstoff wird wie folgt hergestellt : Eine Lösung von 10,8 g Phenylhydrazin in 120 ml Diäthyläther wird mit Eis gekühlt und innerhalb 15 Minuten mit 10,5 g Trifluoressigsäureanhydrid in 25 ml Diäthyläther tropfenweise versetzt. Das Gemisch wird bei 0-5° 15 Minuten, dann bei Zimmertemperatur 2 Stunden gerührt und nachher filtriert. Das Filtrat wird mit Wasser gewaschen, getrocknet, eingedampft und der Rückstand aus Diäthyläther-n-Heptan kristallisiert. Man erhält das β-Trifluoracetyl-phenylhydrazin, welches bei 119-121° schmilzt.

Ein Gemisch von 1,5 g der letztgenannten Verbindung, 100 ml Tetrahydrofuran und 0,06 g Lithiumhydrid wird unter Feuchtigkeitsausschluss 5 Stunden bei Zimmertemperatur gerührt, um eine Lösung zu erhalten. Getrennt rührt man 1,9 g 4-Chlor-3-chlorcarbonyl-chinolinhydrochlorid in 100 ml Tetrahydrofuran mit 0,06 g Lithiumhydrid unter Feuchtigkeitsausschluss eine Minute bei 10° und diese Lösung wird zu der vorhergenannten in Portionen von 10 ml gegeben. Das Reaktionsgemisch wird bei Zimmertemperatur 18 Stunden gerührt, dann 8 Stunden unter Rückfluss gekocht und unter vermindertem Druck konzentriert. Man erhält das 4-Chlor-chinolin-3-(N-phenyl-N-trifluoracetamido)-carboxamid, welches ohne weitere Reinigung verwendet wird.

## Beispiel 20

Ein Gemisch von 0,211 g 4-(0-Methylhydroxylamino)-chinolin-3-(N-p-chlorphenyl)-carboxamid und 15 ml eutektischem Diphenyläther-Biphenyl wird in einer Stickstoffatmosphäre 2 Stunden auf 240° erhitzt. Das Gemisch wird auf Zimmertemperatur gekühlt, mit 150 ml Petroläther verdünnt und der erhaltene Niederschlag abgetrennt. Dieser wird mit Petroläther gewaschen, mit 15 ml Diäthyläther und 3 ml 2-normaler wässeriger Natriumhydroxydlösung eine Stunde gerührt, vom festen Material abfiltriert und die Phasen des Filtrats werden getrennt. Die wässerige Phase wird mit 0,32 g Ammoniumchlorid behandelt. Man erhält einen gelben Niederschlag, der abgetrennt und aus Aethanol umkristallisiert wird. Man erhält das 2-(p-Chlorphenyl)-pyrazolo [4,3-c] chinolin-3(5H)-on, welches bei 327° schmilzt. Das Produkt ist mit demjenigen des Beispiels 1 identisch.

Der Ausgangsstoff wird wie folgt hergestellt : Ein Gemisch von 11,62 g 4-Hydroxy-chinolin-3-carbonsäure [M. Hamana et al., Chem. Pharm. Bull., *26*, 3856 (1978)], 7,84 g p-Chloranilin, 17,59 g 1-Aethoxycarbonyl-2-äthoxy-1,2-dihydrochinolin und 150 ml Dimethylformamid wird 2 Stunden auf 60-70° erhitzt, um eine klare Lösung zu erhalten. Diese wird gekühlt, filtriert und in einem Rotationsverdampfer eingedampft. Der Rückstand wird in Diäthyläther trituriert, filtriert und der Niederschlag mit Diäthyläther gewaschen. Man erhält ein Gemisch, welches den Rest der als Ausgangsstoff verwendeten Säure enthält. Das Gemisch wird mit 100 ml 2-normaler wässeriger Natriumhydroxydlösung 1,5 Stunden gerührt, filtriert, mit Wasser gewaschen und getrocknet. Man erhält das 4-Hydroxychinolin-3-(N-p-chlorphenyl)-carboxamid, welches im IR-Spektrum Banden bei 3 450, 3 260 und 3 210 $cm^{-1}$ zeigt.

Ein Gemisch von 1 g der letztgenannten Verbindung und 25 ml Phosphoroxychlorid wird 3 Stunden auf 80° erhitzt, um eine klare Lösung zu erhalten. Diese wird eingedampft, der Rückstand mit 400 ml eines 1 : 1-Gemisches von Eis und 2-normaler wässeriger Natriumhydroxydlösung behandelt, mit 200 ml Dichlormethan gerührt, filtriert und die Schichten werden getrennt. Die organische Phase wird getrocknet und eingedampft. Man erhält das 4-Chlorchinolin-3-(N-p-chlorphenyl)-carboxamid, welches bei 229-234° schmilzt. (Diese Verbindung kann auch so hergestellt werden, dass man zuerst die genannte Säure mit Phosphoroxchlorid und dann das erhaltene Dichlorid mit dem genannten Anilin behandelt).

Ein Gemisch von 0,5 g der letztgenannten Verbindung, 1 g 0-Methylhydroxylamin-hydrochlorid und 1,65 g Diisopropyläthylamin wird in einem kleinen Druckgefäss 18 Stunden auf 100° erhitzt. Das abgekühlte Gemisch wird mit Wasser trituriert, in Tetrahydrofuran gelöst, getrocknet, eingedampft und der Rückstand aus Methanol umkristallisiert. Man erhält das 4-(0-Methylhydroxylamino)-chinolin-3-(N-p-chlorphenyl)-carboxamid, welches bei 210-212° schmilzt.

## Beispiel 21

Eine Lösung von 308 mg 1-(p-Chlorphenyl)-4-hydroxymethylen-3-(o-nitrophenyl)-4,5-dihydropyrazol-5-on in 80 ml Tetrahydrofuran wird über 50 mg 5 %igem Platin-auf-Kohle-Katalysator bei Zimmertemperatur und 3 Atmosphären eine halbe Stunde hydriert. Das Gemisch wird filtriert, das Filtrat eingedampft und der Rückstand in 150 ml Toluol aufgenommen. Die Lösung wird mit 0,1 ml konz. Chlorwasserstoffsäure versetzt und das Gemisch unter Verwendung eines Wasserabscheiders 18 Stunden unter Rückfluss gekocht. Das Gemisch wird gekühlt, der erhaltene Niederschlag abfiltriert und durch präparative Dünnschichtchromatographie auf Silicagel gereinigt. Als Lösungsmittel verwendet man Essigsäureäthylester : Aethanol : konz. Ammoniumhydroxyd (17 : 3 : 3). Man erhält das 2-(p-Chlorphenyl)-pyrazolo [4,3-c] chinolin-3(5H)-on, welches bei 327° schmilzt. Das Produkt ist mit demjenigen des Beispiels 1 identisch.

Der Ausgangsstoff wird wie folgt hergestellt : Eine Lösung von 39,6 g Malonsäure-monoäthylester, 50 mg 2,2-Bipyridyl (Indikator) und 650 ml Tetrahydrofuran wird auf – 70° gekühlt, und dann unter Rühren in einer Stickstoffatmosphäre mit 305 ml 1,97-molaren n-Butyl-lithium in Hexan langsam versetzt. Man lässt die Temperatur gegen das Ende der Zugabe, nachdem die rosarote Farbe des Indikators bestehen bleibt, auf ungefähr –5° steigen. Das Gemisch wird wieder auf – 65° gekühlt und eine Lösung von 31,7 g o-Nitrobenzoylchlorid in 50 ml Tetrahydrofuran tropfenweise, innerhalb 10 Minuten, zugegeben. Das erhaltene Gemisch wird bei Zimmertemperatur 1 Stunde gerührt und dann auf ein Gemisch von 650 ml 1-normaler Chlorwasserstoffsäure und 1 100 ml Diäthyläther gegossen. Die organische Schicht wird abgetrennt, nacheinander mit 350 ml gesättigter, wässeriger Natriumhydrogencarbonatlösung, 400 ml Wasser und 200 ml gesättigter wässeriger Natriumchloridlösung gewaschen, getrocknet und eingedampft. Man erhält den 2-(o-Nitrobenzoyl)-essigsäureäthylester, als ein farbloses Oel.

Eine Lösung von 3,55 g der letztgenannten Verbindung und 1,7 g p-Chlorphenylhydrazin in 65 ml Toluol wird unter Verwendung eines Wasserabscheiders 3 Stunden unter Rückfluss gekocht. Das Gemisch wird eingedampft und der Rückstand auf Silicagel chromatographiert und mit 15 %igem Essigsäureäthylester in Toluol erhitzt. Man erhält das 1-(p-Chlorphenyl)-3-(o-nitrophenyl)-4,5-dihydropyrozol-5-on, welches bei 138-141° schmilzt.

Man rührt 0,7 g der letztgenannten Verbindung in 10 ml Dimethylformamid-dimethylacetal bei Zimmertemperatur 18 Stunden. Das dunkle Reaktionsgemisch wird auf Eiswasser gegossen, der Niederschlag abgenutscht, in Essigsäureäthylester aufgenommen, mit Wasser gewaschen, getrocknet und eingedampft. Man erhält das 1-(p-Chlorphenyl)-4-dimethylaminomethylen-3-o-nitrophenyl-4,5-dihydropyrazol-5-on, welches bei 208-210° unter Zersetzung schmilzt.

Man rührt 2,5 g der letztgenannten Verbindung in ein Gemisch von 20 ml Tetrahydrofuran und 20 ml 20 %iger wässeriger. Chlorwasserstoffsäure zuerst bei 60° 3 Stunden und dann bei Zimmertemperatur 18 Stunden. Das Gemisch wird mit gesättigter wässeriger Natriumchloridlösung verdünnt, der Niederschlag abgetrennt und mit Essigsäureäthylester gewaschen. Man erhält das 1-(p-Chlorphenyl)-4-hydroxymethylen-3-(o-nitrophenyl)-4,5-dihydropyrazol-1-5-on, welches bei 155-157° schmilzt.

## Beispiel 22

Ein Gemisch von 650 mg 1-(p-Chlorphenyl)-2-methyl-4-anilinomethyliden-pyrazolidin-3,5-dion, 2 g Polyphosphorsäureäthylester und 10 ml 1,1,2,2-Tetrachloräthan wird 24 Stunden unter Rückfluss gekocht. Die Lösung wird auf 10 ml 2-normaler wässeriger Natriumhydroxydlösung gegossen und die organische Schicht auf Silicagelplatten chromatographiert und mit Toluol : Aethanol : konz. Ammoniumhydroxyd (80 : 20 : 1) eluiert. Man erhält das 1-Methyl-2-(p-chlorphenyl)-pyrazolo[4,3-c]chinolin-3-on mit einem Rf-Wert von 0,48. Verwendet man als Eluierungsmittel Dichlormethan : Methanol (19 : 1), so hat die genannte Verbindung einen Rf-Wert von 0,33. Das Produkt ist mit demjenigen des Beispiels 4 identisch.

Der Ausgangsstoff wird wie folgt hergestellt : Man gibt 20 mg Malonsäure-diäthylester zu 5,8 g Natriummetall, welches in 100 ml absolutem Aethanol gelöst ist. Das Gemisch wird 15 Minuten gerührt, mit 17,8 g p-Chlorphenylhydrazin versetzt und dann der überschüssige Aethanol abgesaugt. Der Rückstand wird bei 110 bis 120° 4,5 Stunden erhitzt, dann mit 500 ml Eiswasser abgeschreckt. Das erhaltene Gemisch wird zweimal mit Diäthyläther gewaschen und die wässerige Schicht mit konz. Chlorwasserstoffsäure auf den pH-Wert 2 eingestellt. Das erhaltene feste Material wird aus Toluol umkristallisiert. Man erhält das 1-(p-Chlorphenyl)-pyrazolidin-3,5-dion, welches bei 189-193° schmilzt.

Ein Gemisch von 2 g der letztgenannten Verbindung, 2,81 g Orthoameisensäure-triäthylester, 0,97 g Anilin und 30 ml Aethanol wird 16 Stunden unter Rückfluss gekocht. Das Reaktionsgemisch wird gekühlt, filtriert und der Niederschlag mit Wasser gewaschen. Man erhält das 1-(p-Chlorphenyl)-4-anilinomethyliden-pyrazolidin-3,5-on, welches bei 288-290° schmilzt.

Ein Gemisch von 500 mg der letztgenannten Verbindung, 15 mg Lithiumhydrid und 2 ml Dimethylformamid wird 3 Stunden auf 70° erhitzt. Die Lösung wird dann auf 5° gekühlt und mit 700 mg Methyljodid versetzt. Das Gemisch wird 16 Stunden bei Zimmertemperatur gerührt und eingedampft. Der Rückstand wird in Wasser und Dichlormethan aufgenommen, die organische Phase abgetrennt, getrocknet und eingedampft. Man erhält das 1-(p-Chlorphenyl)-2-methyl-4-anilinomethyliden-pyrazolidin-3,5-dion, welches NMR-Banden bei 3,14, 7,47, 8,42, 10,92 und 10,98 ppm zeigt.

## Beispiel 23

Eine Lösung von 50 mg 1-(p-Chlorphenyl)-3-(o-formylaminophenyl)-4,5-dihydropyrazol-5-on in 10 ml Dichlormethan wird eingedampft, wobei an der inneren Kolbenwand ein dünner Film zurückbleibt. Dieser wird 30 Minuten in einem langsamen Stickstoffstrom auf 190-200° erhitzt. Das Reaktionsprodukt wird auf Silicagelplatten chromatographiert und mit Essigsäureäthylester : Aethanol : konz. Ammoniumhydroxyd (17 : 3 : 3) eluiert. Man erhält das 2-(p-Chlorphenyl)-pyrazolo[4,3-c]chinolin-3(5H)-on, welches einen Rf-Wert von 0,16 aufweist. Verwendet man 5 % Methanol in Dichlormethan, so hat die Verbindung einen Rf-Wert von 0,07. Mit Toluol : Aethanol : konz. Ammoniumhydroxyd (70 : 30 3) ist der Rf-Wert 0,32.

Der Ausgangsstoff wird wie folgt hergestellt : In 100 ml Aethanol hydriert man über 200 mg 5 %igem

Platin-auf-Kohle-Katalysator 2 g 1-(p-Chlorphenyl)-3-(o-nitrophenyl)-4,5-dihydropyrazol-5-on bei Zimmertemperatur und 3 Atmosphären. Da sich das Produkt aus dem Gemisch gleich nach seiner Bildung auskristallisiert, verdünnt man das Reaktionsgemisch mit Dichlormethan, um das kristalline Produkt aufzulösen. Das Gemisch wird filtriert, das Filtrat eingedampft und der getrocknete Rückstand aus Aethanol umkristallisiert. Man erhält das 3-(o-Aminophenyl)-1-(p-Chlorphenyl)-4,5-dihydropyrazol-5-on, welches bei 199-201° schmilzt.

Man gibt in einer Stickstoffatmosphäre, unter Eiskühlung und Rühren 0,3 g der letztgenannten Verbindung zu 10 ml 97 %iger Ameisensäure, wobei eine farblose Lösung entsteht. Diese wird mit 1 ml Essigsäureanhydrid versetzt, über Nacht bei Zimmertemperatur gerührt und dann in 300 ml gesättigter wässeriger Natriumchloridlösung gegossen. Der farblose Niederschlag wird abgetrennt, in 200 ml Diäthyläther gelöst, getrocknet und eingedampft. Man erhält das 1-(p-Chlorphenyl)-3-(o-formylaminophenyl)-4,5-dihydropyrazol-5-on, welches bei 170-172° schmilzt.

## Beispiel 24

Gemäss den in den vorhergehenden Beispielen, insbesondere in den Beispielen 1 und 8 bis 11, illustrierten Methoden werden auch die folgenden Verbindungen der Formel II, in welchen $R_1 = R_2 = H$, hergestellt :

| No. | $R^3$ | R | F °C oder IR-Banden $cm^{-1}$ |
|---|---|---|---|
| 1 | 8-$CH_3O$ | 2-Pyridyl | 319-323 |
| 2 | 7-$CF_3$ | 2-Pyridyl | 348-350 |
| 3 | H | 4-$CH_3$-2-pyridyl | 342-344 |
| 4 | H | 5-Cl-2-pyridyl | 886, 835, 828, 797, 778, 756 |
| 5 | H | 2,5-$Cl_2$-phenyl | 337-338 |
| 6 | H | 3,4-$Cl_2$-phenyl | 890, 878, 867, 823, 818, 795 |
| 7 | H | 3,5-$Cl_2$-phenyl | 890, 871, 847, 830, 806, 788 |

Auch die folgenden Verbindungen der Formel III werden analog den in den vorhergehenden Beispielen illustrierten Methoden hergestellt :

| No. | R″ | R′ | F.°C oder IR-Banden $cm^{-1}$ |
|---|---|---|---|
| 8 | H | o-$CH_3$ | 349-350 (HCl-Salz) |
| 9 | H | p-$CH_3$ | 338-340 |
| 10 | H | p-$OCH_3$ | 268-270 |
| 11 | | p-$SCH_3$ | 307-309 |
| 12 | H | p-F | 342-346 |
| 13 | H | m-F | 335-338 |
| 14 | H | o-F | 338-340 |
| 15 | H | o-Cl | 336-339 |
| 16 | H | m-Cl | 336-337 |
| 17 | H | p-Br | 328-330 |
| 18 | H | p-$CF_3$ | 315-320 |
| 19 | H | p-$NO_2$ | 886, 853, 818, 780, 758, 749 |
| 20 | 7-Cl | H | 872, 851, 830, 818, 798, 770 |
| 21 | 7-Cl | p-Cl | 865, 852, 823, 795, 768, 752 |
| 22 | 7-Cl | p-F | 890, 858, 835, 804, 770, 731 |
| 23 | 7-Cl | m-Cl | 333-335 |
| 24 | 8-$CH_3$ | H | 860, 810, 785, 770, 750, 730 |
| 25 | 8-$CH_3$ | p-$CH_3$ | 340-342 |
| 26 | 8-$CH_3$ | p-Cl | 335-337 |
| 27 | 8-$OCH_3$ | H | 321-325 |
| 28 | 8-$OCH_3$ | p-$OCH_3$ | 313-315 |
| 29 | 8-$OCH_3$ | o-F | 344-347 |
| 30 | 8-$OCH_3$ | m-Cl | 324-327 |
| 31 | 8-$OCH_3$ | p-Cl | 347-349 |
| 32 | 8-F | H | 327-328 |
| 33 | 8-F | p-$OCH_3$ | 289-292 |
| 34 | 8-F | p-F | 884, 868, 827, 767, 715, 708 |

Tabelle (Fortsetzung)

| No. | R″ | R′ | F.°C oder IR-Banden $cm^{-1}$ |
|---|---|---|---|
| 35 | 8-F | m-F | 900, 881, 865, 839, 827, 774 |
| 36 | 8-F | o-F | 868, 850, 815, 782, 750, 724 |
| 37 | 8-F | p-Cl | 342-345 |
| 38 | 8-F | m-Cl | 870, 810, 800, 775, 760, 714 |
| 39 | 8-Cl | H | 894, 871, 845, 812, 787, 770 |
| 40 | 8-Cl | o-F | 895, 875, 858, 820, 814, 782 |
| 41 | 8-Cl | m-F | 892, 884, 867, 860, 815, 771 |
| 42 | 8-Cl | p-F | 898, 890, 870, 855, 832, 820 |
| 43 | 8-Cl | o-Cl | 896, 885, 880, 848, 823, 789 |
| 44 | 8-Cl | p-Cl | 890, 842, 820, 806, 782, 768 |
| 45 | 8-Cl | m-Cl | 890, 865, 815, 790, 777, 740 |
| 46 | 8-Cl | p-$NO_2$ | 895, 882, 849, 834, 808, 784 |
| 47 | 8-F | p-$NO_2$ | 896, 884, 869, 860, 828, 821 |
| 48 | 8-$OCH_3$ | p-$NO_2$ | 338-340 |
| 49 | 6-Cl | p-Cl | 825, 806, 762, 736, 720 |
| 50 | 7-$CF_3$ | H | 324-327 |
| 51 | 7-$CF_3$ | p-F | 331-334 |
| 52 | 7-$CF_3$ | m-Cl | 317-320 |
| 53 | 7-$CF_3$ | p-Cl | 890, 854, 826, 800, 770, 756 |

Beispiel 25

Tabletten enthaltend 5 mg des Wirkstoffes.

Zusammensetzung (für 10 000 Tabletten) :

| | |
|---|---|
| 2-(p-Chlorphenyl)-pyrazolo [4,3-c] chinolin-3-(5H)-on | 50 g |
| Milchzucker | 1 157 g |
| Maisstärke | 75 g |
| Polyäthylenglykol 6000 | 75 g |
| Talkpulver | 75 g |
| Magnesiumstearat | 18 g |
| Gereinigtes Wasser | q. s. |

Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 40 ml Wasser suspendiert und die Suspension zur siedenden Lösung von Polyäthylenglykol in 150 ml Wasser gegeben. Die erhaltene Paste wird zu den Pulvern gegeben und gegebenenfalls unter Zugabe einer weiteren Wassermenge granuliert. Das Granulat wird über Nacht bei 35° getrocknet, durch ein Sieb von 1,2 mm Maschenweite getrieben und zu Tabletten von 6,4 mm Durchmesser, welche eine Bruchrille aufweisen, gepresst.

Beispiel 26

Kapseln enthaltend 10 mg des Wirkstoffes.

Zusammensetzung (für 10 000 Kapseln)

| | |
|---|---|
| 2-Phenyl-pyrazolo [4,3-c] chinolin-3(5H)-on | 100 g |
| Milchzucker | 1 800 g |
| Talkpulver | 100 g |

Sämtliche Pulver werden mit einem Sieb von 0,6 mm Maschenweite gesiebt. Dann wird der Wirkstoff zuerst mit dem Talk und darauffolgend mit dem Milchzucker in einem geeigneten Mischer homogenisiert. Kapseln No. 3 werden mit je 200 mg des Gemisches mit einer Füllmaschine gefüllt.

In analoger Weise werden auch Tabletten und Kapseln mit den anderen Verbindungen der Erfindung, z. B. mit denjenigen der vorliegenden Beispiele, hergestellt.

**Ansprüche** (für die Vertragsstaaten : BE, CH, DE, FR, GB, IT, LI, LU, NL, SE)

1. Verbindungen der allgemeinen Formeln I und II

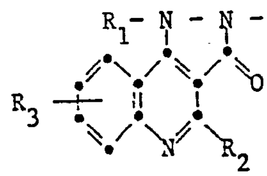 (I) und 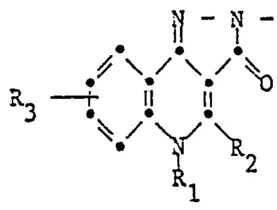 (II)

worin $R_3$ Wasserstoff oder höchstens 3 gleiche oder verschiedene Substituenten der Gruppe $C_1$-$C_7$-Alkyl, $C_1$-$C_7$-Alkoxy, $C_1$-$C_7$-Alkylthio, Hydroxy, Halogen, Trifluormethyl, Nitro, Amino, Mono- oder Di-$C_1$-$C_7$-Alkylamino, Cyan, Carbamoyl oder Carboxy bedeutet, R für unsubstituiertes oder durch höchstens 3 der für $R_3$ angegebenen Reste substituiertes Phenyl, Pyridyl, $C_1$-$C_7$-Alkylpyridyl oder Halogenpyridyl steht, $R_1$ Wasserstoff, $C_1$-$C_7$-Alkyl oder (Hydroxy, Di-$C_1$-$C_7$-Alkylamino oder $R_3$-Phenyl)-$C_1$-$C_7$-Alkyl bedeutet, worin die Hydroxy- oder Aminogruppe vom Ringstickstoffatom durch mindestens zwei Kohlenstoffatome getrennt ist, und $R_2$ für Wasserstoff oder $C_1$-$C_7$-Alkyl steht ; ihre 3-Hydroxy tautomeren Verbindungen ; $C_1$-$C_7$-Alkanoyl-, Carbamoyl-, Mono- oder Di-$C_1$-$C_7$-Alkylcarbamoyl-Derivate der genannten (Hydroxy oder Amino)-(phenyl oder phenylen)-Verbindungen ; oder ihre Salze.

2. Verbindungen nach Anspruch 1, der Formeln I und II, worin $R_3$ Wasserstoff oder höchstens 3 gleiche oder verschiedene Substituenten der Gruppe $C_1$-$C_7$-Alkyl, $C_1$-$C_7$-Alkoxy, $C_1$-$C_7$-Alkylthio, Hydroxy, Halogen, Trifluormethyl, Nitro, Amino, Mono- oder Di-$C_1$-$C_7$-Alkylamino bedeutet, und die anderen Symbole die im Anspruch 1 angegebenen Bedeutungen haben.

3. Verbindungen nach Anspruch 1 der Formeln I und II, worin $R_3$ Wasserstoff oder einen oder zwei Substituenten der Gruppe $C_1$-$C_7$-Alkyl, $C_1$-$C_7$-Alkoxy, $C_1$-$C_7$-Alkylthio, Hydroxy, Halogen, Trifluormethyl, Nitro, Amino, Mono- oder Di-$C_1$-$C_7$-Alkylamino, Cyan, Carbamoyl oder Carboxy bedeutet. R für unsubstituiertes oder durch einen oder zwei der für $R_3$ angegebenen Reste substituiertes Phenyl, Pyridyl, $C_1$-$C_7$-Alkylpyridyl oder Halogenpyridyl steht, $R_1$ Wasserstoff, $C_1$-$C_7$-Alkyl- oder (Hydroxy, Di-$C_1$-$C_7$-Alkylamino oder $R_3$-Phenyl)-$C_1$-$C_7$-Alkyl bedeutet, worin die Hydroxy- oder Aminogruppe vom Ringstickstoffatom durch mindestens 2 Kohlenstoffatome getrennt ist, und $R_2$ für Wasserstoff oder $C_1$-$C_7$-Alkyl steht.

4. Verbindungen nach Anspruch 1 der Formeln I und II, worin $R_3$ Wasserstoff oder einen oder zwei Substituenten der Gruppe Niederalkyl, Niederalkoxy, Niederalkylthio, Hydroxy, Halogen, Trifluormethyl, Nitro, Amino- Mono- oder Di-niederalkylamino substituiert ist und die anderen Symbole die im Anspruch 3 angegebenen Bedeutungen haben.

5. Verbindungen nach Anspruch 1, welche der Formel III

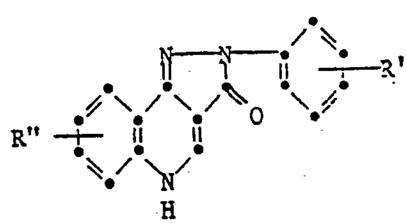 (III)

entsprechen, worin R″ Wasserstoff, Alkyl oder Alkoxy mit jeweils 4 Kohlenstoffatomen, Hydroxy, Fluor, Chlor, Brom oder Trifluormethyl bedeutet, R′ für Wasserstoff, Alkyl oder Alkoxy mit je 4 Kohlenstoffatomen, Hydroxy, Fluor, Chlor, Brom, Trifluormethyl, Nitro, Amino, Monoalkylamino oder Alkylcarbamoylamino mit je höchstens 4 Kohlenstoffatomen, oder Cyan steht, oder ihre Salze.

6. Verbindungen nach Anspruch 1, welche der im Anspruch 5 gezeigten Formel III entsprechen, worin R″ Wasserstoff, Alkyl oder Alkoxy mit jeweils 4 Kohlenstoffatomen, Hydroxy, Fluor, Chlor, Brom oder Trifluormethyl bedeutet, R′ für Wasserstoff, o- oder m-Fluor steht ; oder ihre Salze.

7. 2-(p-Chlorphenyl)-pyrazolo [4,3-c] chinolin-3(5H)-on oder seine Salze.

8. 2-Phenyl-pyrazolo [4,3-c] chinolin-3(5H)-on oder seine Salze.

9. Verbindungen gemäss Anspruch 1, zur Verwendung als psychoaktive Wirkstoffe.

10. Verbindungen gemäss Anspruch 2, zur Verwendung als psychoaktive Wirkstoffe.

11. Pharmazeutische Präparate enthaltend Verbindungen gemäss einem der Ansprüche 1, 3, 5, 8 und 9 oder therapeutisch verwendbare Salze von solchen Verbindungen.

12. Pharmazeutische Präparate enthaltend Verbindungen gemäss einem der Ansprüche 2, 4, 6, 7 und 10 oder therapeutisch verwendbare Salze von solchen Verbindungen.

13. Die Verbindungen der Ansprüche 1, 3, 5, 8 und 9 zur Verwendung als psychoaktive Mittel bei der Behandlung des menschlichen oder tierischen Körpers.

14. Die Verbindungen der Ansprüche 2, 4, 6, 7 und 10 zur Verwendung als psychoaktive Mittel bei der Behandlung des menschlichen oder tierischen Körpers.

15. Verwendung von Verbindungen der Ansprüche 1, 3, 5, 8 und 9 zur Herstellung von pharmazeutischen Präparaten.

16. Verwendung von Verbindungen der Ansprüche 2, 4, 6, 7 und 10 zur Herstellung von pharmazeutischen Präparaten.

17. Verfahren zur Herstellung von im Anspruch 1 definierten 2-Arylpyrazolo [4,3-c] chinolin-3(1 und 5H)-on-Verbindungen, dadurch gekennzeichnet, dass man

a) Verbindungen der Formel IV

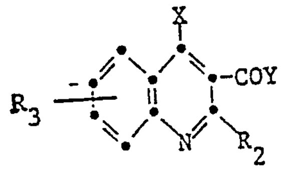

(IV)

worin X —NH—NH—R bedeutet und Y für Hydroxy oder $C_1$-$C_7$-Alkoxy steht ; oder X Halogen bedeutet und Y für $H_2N$—N—R steht ; oder X $C_1$-$C_7$-Alkoxyamino oder Azido bedeutet und Y für NH—R steht, ringschliesst, und, wenn erwünscht, eine erhaltene Verbindung oder ein Alkalimetallsalz davon, mit einem reaktionsfähigen Ester eines Alkohols $R_1$—OH umsetzt, oder

b) Verbindungen der Formel V

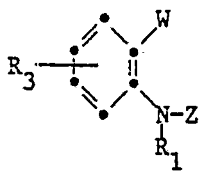

(V)

worin, die beiden Symbole W und $R_1$ Wasserstoff bedeuten und Z für die Gruppierung

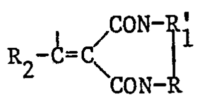

steht, worin $R_1'$ $C_1$-$C_7$-Alkyl bedeutet ; oder W die Gruppierung

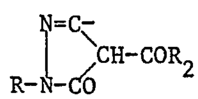

ist und Z für Wasserstoff steht ; oder W die Gruppierung

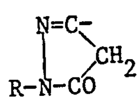

bedeutet und Z für $R_2$—CO oder $R_1$—N—Z zusammengenommen für Isocyano steht, kondensiert, und, wenn erwünscht, eine erhaltene Verbindung in eine andere Verbindung der Erfindung umwandelt, und/oder, wenn erwünscht, eine erhaltene Verbindung in ihr Salz oder ein erhaltenes Salz in die freie Verbindung oder in ein anderes Salz überführt, und/oder, wenn erwünscht, ein erhaltenes Gemisch von Isomeren in die einzelnen Isomeren auftrennt.

18. Die nach dem Verfahren des Anspruchs 17 erhältlichen Verbindungen.

**Ansprüche** (für den Vertragsstaat AT)

1. Verfahren zur Herstellung von neuen 2-Aryl-pyrazolo [4,3-c] chinolin-3(1 und 5H)-on-Verbindungen der allgemeinen Formeln I und II

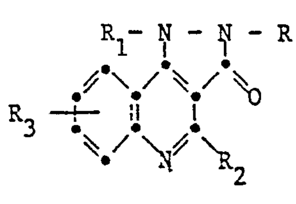

(I) und

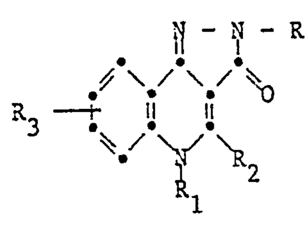

(II)

worin $R_3$ Wasserstoff oder höchstens 3 gleiche oder verschiedene Substituenten der Gruppe $C_1$-$C_7$-Alkyl, $C_1$-$C_7$-Alkoxy, $C_1$-$C_7$-Alkylthio, Hydroxy, Halogen, Trifluormethyl, Nitro, Amino, Mono- oder Di-$C_1$-$C_7$-Alkylamino, Cyan, Carbamoyl oder Carboxy bedeutet, R für unsubstituiertes oder durch höchstens 3 der für $R_3$ angegebenen Reste substituiertes Phenyl, Pyridyl, $C_1$-$C_7$-Alkylpyridyl oder Halogenpyridyl steht, $R_1$ Wasserstoff, $C_1$-$C_7$-Alkyl oder (Hydroxy, Di-$C_1$-$C_7$-Alkylamino oder $R_3$-Phenyl)-$C_1$-$C_7$-Alkyl bedeutet, worin die Hydroxy- oder Aminogruppe vom Ringstickstoffatom durch mindestens zwei Kohlenstoffatome getrennt ist, und $R_2$ für Wasserstoff oder $C_1$-$C_7$-Alkyl steht ; ihre 3-Hydroxy tautomeren Verbindungen ; $C_1$-$C_7$-Alkanoyl-, Carbamoyl-, Mono- oder Di-$C_1$-$C_7$-Alkylcarbamoyl-Derivate der genannten (Hydroxy oder Amino)-(phenyl oder phenylen)-Verbindungen ; oder ihren Salzen, dadurch gekennzeichnet, dass man

a) Verbindungen der Formel IV

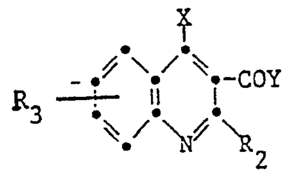

(IV)

worin X —NH—NH—R bedeutet und Y für Hydroxy oder $C_1$-$C_7$-Alkoxy steht ; oder X Halogen bedeutet und Y für $H_2N-\overset{|}{N}-R$ steht ; oder X $C_1$-$C_7$-Alkoxyamino oder Azido bedeutet und Y für NH—R steht, ringschliesst, und, wenn erwünscht, eine erhaltene Verbindung oder ein Alkalimetallsalz davon, mit einem reaktionsfähigen Ester eines Alkohols $R_1$—OH umsetzt, oder

b) Verbindungen der Formel V

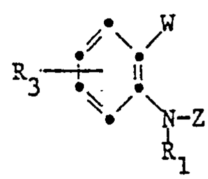

(V)

worin die beiden Symbole W und $R_1$ Wasserstoff bedeuten und Z für die Gruppierung

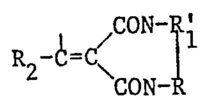

steht, worin $R_1'$ $C_1$-$C_7$-Alkyl bedeutet ; oder W die Gruppierung

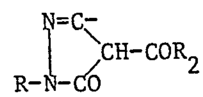

ist und Z für Wasserstoff steht ; oder W die Gruppierung

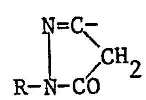

bedeutet und Z für $R_2$—CO oder $R_1-\overset{|}{N}-Z$ zusammengenommen für Isocyano steht, kondensiert, und, wenn erwünscht, eine erhaltene Verbindung in eine andere Verbindung der Erfindung umwandelt, und/oder, wenn erwünscht, eine erhaltene Verbindung in ihr Salz oder ein erhaltenes Salz in die freie Verbindung oder in ein anderes Salz überführt, und/oder, wenn erwünscht, ein erhaltenes Gemisch von Isomeren in die einzelnen Isomeren auftrennt.

2. Verfahren nach Anspruch 1, dadurch gekennzeichnet, dass man eine erhaltene Verbindung in eine andere Verbindung der Erfindung umwandelt, indem man eine Verbindung, in welcher $R_1$ Wasserstoff bedeutet, in 1-Stellung durch Umsetzung mit einem reaktionsfähigen Ester des Alkohols $R_1$—OH substituiert, und/oder eine erhaltene Verbindung der Formel II, worin $R_1$ ein Alkalimetallatom bedeutet, in 5-Stellung durch Umsetzung mit einem reaktionsfähigen Ester des Alkohols $R_1$—OH substituiert, und/oder eine erhaltene $C_1$-$C_7$-Alkoxy-Verbindung zu dem entsprechenden Phenol hydrolysiert, und/oder, eine erhaltene Nitro-Verbindung zu dem entsprechenden Amin reduziert, und/oder, eine erhaltene Amino-Verbindung alkyliert oder acyliert, wobei die Acylgruppen im Anspruch 1 angegeben sind, und/oder, ein erhaltenes Nitril in das entsprechende Amid umwandelt oder zu der Carboxygruppe hydrolysiert.

3. Verfahren nach einem der Ansprüche 1 oder 2, dadurch gekennzeichnet, dass man Verbindungen der in Anspruch 1 gezeigten Formeln I und II, worin $R_3$ Wasserstoff oder einen oder zwei Substituenten der Gruppe $C_1$-$C_7$-Alkyl, $C_1$-$C_7$-Alkoxy, $C_1$-$C_7$-Alkylthio, Hydroxy, Halogen, Trifluormethyl, Nitro, Amino, Mono- oder Di-$C_1$-$C_7$-Alkylamino, Cyan, Carbamoyl oder Carboxy substituiert ist, R für unsubstituiertes oder durch einen oder zwei der für $R_3$ angegebenen Reste substituiertes Phenyl, Pyridyl, $C_1$-$C_7$-Alkylpyridyl oder Halogenpyridyl steht, $R_1$ Wasserstoff, $C_1$-$C_7$-Alkyl oder (Hydroxy, Di-$C_1$-$C_7$-Alkylamino oder $R_3$-Phenyl)-$C_1$-$C_7$-Alkyl bedeutet, worin die Hydroxy- oder Aminogruppe vom Ringstickstoffatom durch mindestens 2 Kohlenstoffatome getrennt ist, und $R_2$ für Wasserstoff oder $C_1$-$C_7$-Alkyl steht ; $C_1$-$C_7$-Alkanoyl-, Carbamoyl-, Mono- oder Di-$C_1$-$C_7$-Alkylcarbamoyl-Derivate der genannten (Hydroxy oder Amino)-(phenyl oder phenylen)-Verbindungen ; oder ihre Salze herstellt.

4. Verfahren nach einem der Ansprüche 1 und 2, dadurch gekennzeichnet, dass man Verbindungen der Formel III

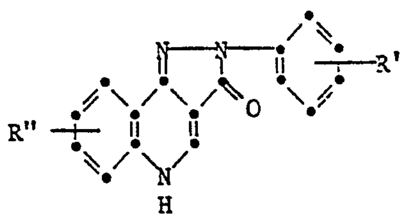

(III)

worin R″ Wasserstoff, Alkyl oder Alkoxy mit jeweils 4 Kohlenstoffatomen, Hydroxy, Fluor, Chlor, Brom oder Trifluormethyl bedeutet, R′ für Wasserstoff, Alkyl oder Alkoxy mit je 4 Kohlenstoffatomen, Hydroxy, Fluor, Chlor, Brom, Trifluormethyl, Nitro, Amino, Monoalkylamino oder Alkylcarbamoylamino mit je höchstens 4 Kohlenstoffatomen, oder Cyan steht, oder ihre Salze herstellt.

5. Verfahren nach Anspruch 1, dadurch gekennzeichnet, dass man 2-(p-Chlorphenyl)-pyrazolo [4,3-c] chinolin-3(5H)-on oder seine Salze herstellt.

6. Verfahren nach Anspruch 1, dadurch gekennzeichnet, dass man 2-Phenyl-pyrazolo [4,3-c] chinolin-3(5H)-on oder seine Salze herstellt.

7. Verfahren nach Anspruch 1 zur Herstellung von 2-Arylpyrazolo [4,3-c] chinolin-3(1 und 5H)-on-Verbindungen der im Anspruch 1 gezeigten Formeln I und II, worin $R_3$ Wasserstoff oder höchstens 3 gleiche oder verschiedene Substituenten der Gruppe $C_1$-$C_7$-Alkyl, $C_1$-$C_7$-Alkoxy, $C_1$-$C_7$-Alkylthio, Hydroxy, Halogen, Trifluormethyl, Nitro, Amino, Mono- oder Di-$C_1$-$C_7$-Alkylamino bedeutet, R für unsubstituiertes oder durch höchstens 3 der für $R_3$ angegebenen Reste substituiertes Phenyl, Pyridyl, $C_1$-$C_7$-Alkylpyridyl oder Halogenpyridyl steht, $R_1$ Wasserstoff, $C_1$-$C_7$-Alkyl oder (Hydroxy, Di-$C_1$-$C_7$-Alkylamino oder $R_3$-Phenyl)-$C_1$-$C_7$-Alkyl bedeutet, worin die Hydroxy- oder Aminogruppe vom Ringstickstoffatom durch mindestens zwei Kohlenstoffatome getrennt ist, und $R_2$ für Wasserstoff oder $C_1$-$C_7$-Alkyl steht ; sowie ihre 3-Hydroxy tautomeren Verbindungen ; und $C_1$-$C_7$-Alkanoyl-, Carbamoyl-, Mono- oder Di-$C_1$-$C_7$-Alkylcarbamoyl-Derivate der genannten (Hydroxy oder Amino)-(phenyl oder phenylen)-Verbindungen ; oder ihre Salze, dadurch gekennzeichnet, dass man

a) Verbindungen der Formel IV

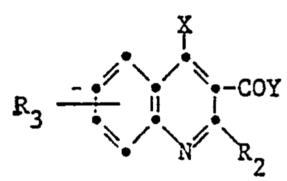

(IV)

worin X —NH—NH—R bedeutet und Y für Hydroxy oder Niederalkoxy steht ; oder X Halogen bedeutet und Y für $H_2$—N—N—R steht, ringschliesst, und, wenn erwünscht, eine erhaltene Verbindung oder ein Alkalimetallsalz davon, mit einem reaktionsfähigen Ester eines Alkohols $R_1$—OH umsetzt, oder

b) Verbindungen der Formel V

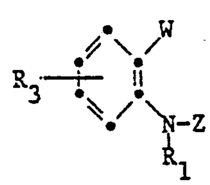

(V)

worin W die Gruppierung

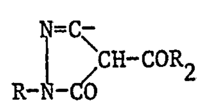

ist und Z für Wasserstoff steht, kondensiert, und, wenn erwünscht, eine erhaltene Verbindung in eine andere Verbindung der Erfindung umwandelt, und/oder, wenn erwünscht, eine erhaltene Verbindung in ihr Salz oder ein erhaltenes Salz in die freie Verbindung oder in ein anderes Salz überführt, und/oder, wenn erwünscht, ein erhaltenes Gemisch von Isomeren in die einzelnen Isomeren.

8. Verfahren nach Anspruch 7, dadurch gekennzeichnet, dass man eine erhaltene Verbindung in eine andere Verbindung der Erfindung umwandelt, indem man eine Verbindung, in welcher $R_1$ Wasserstoff bedeutet, in 1-Stellung durch Umsetzung mit einem reaktionsfähigen Ester des Alkohols $R_1$—OH substituiert, und/oder, eine erhaltene Verbindung der Formel II, worin $R_1$ ein Alkalimetallatom bedeutet, in 5-Stellung durch Umsetzung mit einem reaktions fähigen Ester des Alkohols $R_1$-OH substituiert, und/oder eine erhaltene $C_1$-$C_7$-Alkoxy-Verbindung zu dem entsprechenden Phenol hydrolysiert, und/oder, eine erhaltene Nitro-Verbindung zu dem entsprechenden Amin reduziert, und/oder eine erhaltene Amino-Verbindung alkyliert oder acyliert, wobei die Acylgruppen im Anspruch 7 angegeben sind.

9. Verfahren nach einem der Ansprüche 7 und 8, dadurch gekennzeichnet, dass man Verbindungen der im Anspruch 1 angegebenen Formeln I und II, worin $R_3$ Wasserstoff oder einen oder zwei Substituenten der Gruppe $C_1$-$C_7$-Alkyl, $C_1$-$C_7$-Alkoxy, $C_1$-$C_7$-Alkylthio, Hydroxy, Halogen, Trifluormethyl, Nitro, Amino, Mono- oder Di-$C_1$-$C_7$-Alkylamino bedeutet, R für unsubstituiertes oder durch einen oder zwei der für $R_3$ angegebenen Reste substituiertes Phenyl, Pyridyl, $C_1$-$C_7$-Alkylpyridyl oder Halogenpyridyl steht, $R_1$ Wasserstoff, $C_1$-$C_7$-Alkyl oder (Hydroxy, Di-$C_1$-$C_7$-Alkylamino- oder $R_3$-Phenyl)-$C_1$-$C_7$-Alkyl bedeutet, worin die Hydroxy- oder Aminogruppe vom Ringstickstoffatom durch mindestens 2 Kohlenstoffatome getrennt ist, und $R_2$ für Wasserstoff oder $C_1$-$C_7$-Alkyl steht ; $C_1$-$C_7$-Alkanoyl-, Carbamoyl-, Mono- oder Di-$C_1$-$C_7$-Alkylcarbamoyl-Derivate der genannten (Hydroxy oder Amino)-(phenyl oder phenylen)-Verbindungen ; oder ihre Salze herstellt.

10. Verfahren nach einem der Ansprüche 7 und 8, dadurch gekennzeichnet, dass man Verbindungen der in Anspruch 4 gezeigten Formel III, worin R″ Wasserstoff, Alkyl oder Alkoxy mit jeweils 4 Kohlenstoffatomen, Hydroxy, Fluor, Chlor, Brom oder Trifluormethyl bedeutet, R′ für Wasserstoff, o- oder m-Fluor steht ; oder ihre Salze herstellt.

11. Verfahren nach Anspruch 7, dadurch gekennzeichnet, dass man 2-(p-Chlorphenyl)-pyrazolo [4,3-c] chinolin-3(5H)-on oder seine Salze herstellt.

12. Verfahren zur Herstellung eines pharmazeutischen Präparates, gekennzeichnet durch die Verarbeitung eines erfindungsgemässen Wirkstoffs nach einem der Ansprüche 1 bis 6, mit einem pharmazeutischen Trägermaterial.

13. Verfahren zur Herstellung eines pharmazeutischen Präparates, gekennzeichnet durch die Verarbeitung eines erfindungsgemässen Wirkstoffs nach einem der Ansprüche 7 bis 10, mit einem pharmazeutischen Trägermaterial.

**Claims** (for the Contracting States : BE, CH, DE, FR, GB, IT, LI, LU, NL, SE)

1. A Compound of the general formulae I and II

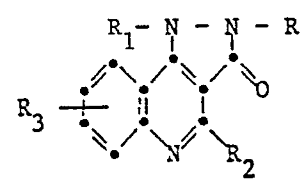 (I) and 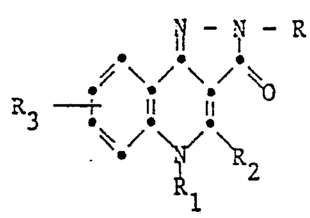 (II)

wherein $R_3$ is hydrogen or not more than 3 identical or different members selected from $C_1$-$C_7$-alkyl, $C_1$-$C_7$-alkoxy, $C_1$-$C_7$-alkylthio, hydroxy, halogen, trifluoromethyl, nitro, amino, mono- or di-$C_1$-$C_7$-alkylamino, cyano, carbamoyl and carboxy ; R is phenyl or phenyl substituted by not more than 3 of the radicals as defined for $R_3$, pyridyl, $C_1$-$C_7$-alkylpyridyl, or halopyridyl ; $R_1$ is hydrogen, $C_1$-$C_7$-alkyl or (hydroxy, di-$C_1$-$C_7$-alkyl-mino or $R_3$-phenyl)-$C_1$-$C_7$-alkyl, wherein the hydroxy or amino group is separated from the ring nitrogen atom by at least 2 carbon atoms, and $R_2$ is hydrogen or $C_1$-$C_7$-alkyl ; their 3-hydroxy-tautomers ; $C_1$-$C_7$-alkanoyl, carbamoyl, mono- or di-$C_1$-$C_7$-alkylcarbamoyl derivatives of said (hydroxy or amino)-(phenyl or phenylene) compounds ; or salts thereof.

2. A compound according to claim 1 of the formulae I and II, wherein $R_3$ is hydrogen or not more than 3 identical or different members selected from $C_1$-$C_7$-alkyl, $C_1$-$C_7$-alkoxy, $C_1$-$C_7$-alkylthio, hydroxy, halogen, trifluoromethyl, nitro, amino or mono- or di-$C_1$-$C_7$-alkylamino ; and the other symbols have the meanings given in claim 1.

3. A compound according to claim 1 of the general formulae I and II, wherein $R_3$ is hydrogen or one or two members selected from $C_1$-$C_7$-alkyl, $C_1$-$C_7$-alkoxy, $C_1$-$C_7$-alkylthio, hydroxy, halogen, trifluoromethyl, nitro, amino, mono- or di-$C_1$-$C_7$-alkylamino, cyano, carbamoyl and carboxy ; R is phenyl or phenyl substituted by one or two of the substituents as defined for $R_3$, pyridyl, $C_1$-$C_7$-alkylpyridyl or

halopyridyl ; $R_1$ is hydrogen, $C_1$-$C_7$-alkyl or (hydroxy, di-$C_1$-$C_7$-alkylamino or $R_3$-phenyl)-$C_1$-$C_7$-alkyl, wherein the hydroxy or amino group is separated from the ring-nitrogen atom by at least 2 carbon atoms ; and $R_2$ is hydrogen or $C_1$-$C_7$-alkyl.

4. A compound according to claim 1 of the formulae I and II, wherein $R_3$ is hydrogen or one or two members selected from lower alkyl, lower alkoxy, lower alkylthio, hydroxy, halogen, trifluoromethyl, nitro, amino, mono- or di-lower alkylamino ; and the other symbols have the meanings given in claim 3.

5. A compound according to claim 1 of the formula III

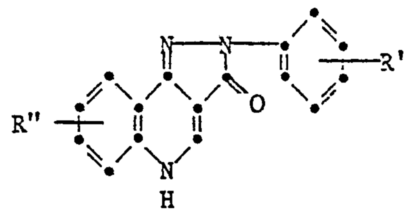

(III)

wherein R″ is hydrogen, alkyl or alkoxy, each containing 4 carbon atoms, hydroxy, fluorine, chlorine, bromine or trifluoromethyl ; and R′ is hydrogen, alkyl or alkoxy each containing 4 carbon atoms, hydroxy, fluorine, chlorine, bromine, trifluoromethyl, nitro, amino, monoalkylamino or alkylcarbamoylamino, each containing 4 carbon atoms, or cyano ; or a salt thereof.

6. A compound according to claim 1 of the formula III as indicated in claim 5, wherein R″ is hydrogen, alkyl or alkoxy, each containing 4 carbon atoms, hydroxy, fluorine, chlorine, bromine or trifluoromethyl, R′ is hydrogen, o- or -m-fluorine, or a salt thereof.

7. 2-(p-Chlorophenyl)-pyrazolo [4,3-c] quinolin-3(5H)-one or a salt thereof.

8. 2-Phenyl-pyrazolo [3,4-c] quinolin-3(5H)-one or a salt thereof.

9. A compound according to claim 1 for use as psychoactive drug.

10. A compound according to claim 2 for use as psychoactive drug.

11. A pharmaceutical preparation containing a compound according to any one of claims 1, 3, 5, 8 and 9, or a therapeutically acceptable salt thereof.

12. A pharmaceutical preparation containing a compound according to any of claims 2, 4, 6, 7 and 10, or a therapetically acceptable salt thereof.

13. A compound of any one of claims 1, 3, 5, 8 and 9 for use as a psychoactive drug for treating the human or animal body.

14. A compound of any one of claims 2, 4, 6, 7 and 10 for use as a psychoactive drug for treating the human of animal body.

15. Use of a compound according to any one of claims 1, 3, 5, 8 or 9 for the manufacture of pharmaceutical preparations.

16. Use of a compound according to any one of claims 2, 4, 6, 7 or 10 for the manufacture of pharmaceutical preparations.

17. A process for the manufacture of a 2-aryl-pyrazolo [4,3-c] quinolin-3-(1 or 5H)-one as defined in claim 1, which comprises

a) cyclising a compound of the formula IV

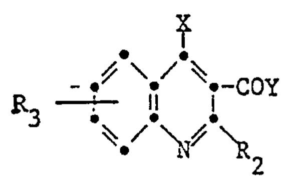

(IV)

wherein X is —NH—NH—R and Y is hydroxy of $C_1$-$C_7$-alkoxy ; or X is halogen and Y is $H_2N$—R ; or X is $C_1$-$C_7$-alkoxyamino or azido, and Y is NH—R, and, if desired, reacting a resulting compound, or an alkali metal salt thereof, with a reactive ester of the alcohol $R_1$—OH, or

b) condensing a compound of the formula V

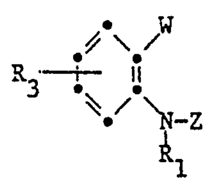

(V)

wherein W and $R_1$ are both hydrogen, Z is

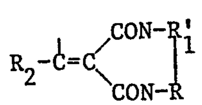

and $R_1'$ is $C_1$-$C_7$-alkyl ; or W is

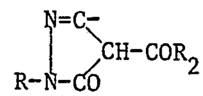

and Z is hydrogen ; or W is

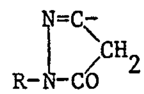

and Z is $R_2$—CO, or $R_1$—N—Z together is isocyano ; and, if desired, converting a resulting compound into another compound of the invention, and/or, if desired, converting a resulting compound into a salt thereof, or a resulting salt into the corresponding free compound or into another salt, and/or, if desired, separating a mixture of isomers obtained into the individual isomers.

18. A compound obtainable according to the process of claim 17.

**Claims** (for the Contracting State AT)

1. A process for the preparation of a 2-aryl-pyrazolo [4,3-c] quinolin-3-(1 or 5H)-one of the general formulae I and II

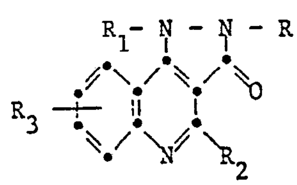 (I) and 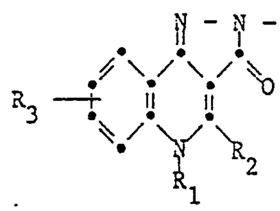 (II)

wherein $R_3$ is hydrogen or not more than 3 identical or different members selected from $C_1$-$C_7$-alkyl, $C_1$-$C_7$-alkoxy, $C_1$-$C_7$-alkylthio, hydroxy, halogen, trifluoromethyl, nitro, amino, mono- or di-$C_1$-$C_7$-alkylamino, cyano, carbamoyl and carboxy ; R is phenyl or phenyl substitued by not more than 3 of the radicals as defined for $R_3$, pyridyl, $C_1$-$C_7$-alkylpyridyl, or halopyridyl ; $R_1$ is hydrogen, $C_1$-$C_7$-alkyl or (hydroxy, di-$C_1$-$C_7$-alkylamino or $R_3$-phenyl)-$C_1$-$C_7$-alkyl, wherein the hydroxy or amino group is separated from the ring nitrogen atom by at least 2 carbon atoms, and $R_2$ is hydrogen or $C_1$-$C_7$-alkyl ; their 3-hydroxy-tautomers ; $C_1$-$C_7$-alkanoyl, carbamoyl, mono- or di-$C_1$-$C_7$-alkylcarbamoyl derivatives of said (hydroxy or amino)-(phenyl or phenylene) compounds ; or a salt thereof, which comprises

a) cyclising a compound of the formula IV

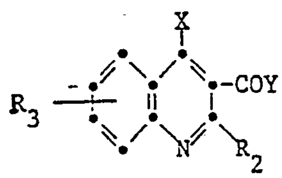 (IV)

wherein X is —NH—NH—R and Y is hydroxy or $C_1$-$C_7$-alkoxy ; or X is halogen and Y is $H_2N$—R ; or X is $C_1$-$C_7$-alkoxyamino or azido, and Y is NH—R, and, if desired, reacting a resulting compound, or an alkali metal salt thereof, with a reactive ester of the alcohol $R_1$—OH, or

b) condensing a compound of the formula V

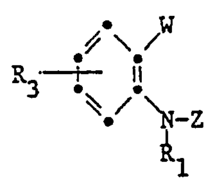 (V)

wherein W and $R_1$ are both hydrogen, Z is

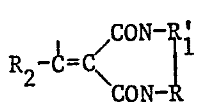

and $R_1'$ is $C_1$-$C_7$-alkyl ; or W is

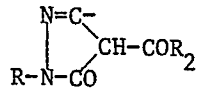

and Z is hydrogen ; or W is

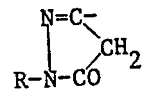

and Z is $R_2$—CO, or $R_1$—N—Z together is isocyano ; and, if desired, converting a resulting compound into another compound of the invention, and/or, if desired, converting a resulting compound into a salt thereof, or a resulting salt into the corresponding free compound or into another salt, and/or, if desired, separating a mixture of isomers obtained into the individual isomers.

2. A process according to claim 1, which comprises converting a resultant compound into another compound of the invention by substituting a compound, in which $R_1$ is hydrogen, in the 1-position by reaction with a reactive ester of the alcohol $R_1$—OH, and/or substituting a resultant compound of the formula II, in which $R_1$ is an alkali metal atom, in the 5-position by reaction with a reactive ester of the alcohol $R_1$—OH, and/or hydrolysing a resultant $C_1$-$C_7$-alkoxy compound to the corresponding phenol, and/or reducing a resultant nitro compound to the corresponding amine, and/or alkylating or acylating a resultant amino compound in which the acyl groups are as indicated in claim 1, and/or converting a resultant nitrile into the corresponding amide of hydrolysing it to the carboxyl group.

3. A process according to either of claims 1 and 2 for the preparation of a compound of the formulae I and II as indicated in claim 1, wherein $R_3$ is hydrogen or one or two members selected from $C_1$-$C_7$-alkyl, $C_1$-$C_7$-alkoxy, $C_1$-$C_7$-alkylthio, hydroxy, halogen, trifluoromethyl, nitro, amino, mono- or di-$C_1$-$C_7$-alkylamino, cyano, carbamoyl and carboxy ; R is phenyl or phenyl substituted by one or two of the substituents as defined for $R_3$, pyridyl, $C_1$-$C_7$-alkylpyridyl or halopyridyl : $R_1$ is hydrogen, $C_1$-$C_7$-alkyl or (hydroxy, di-$C_1$-$C_7$-alkylamino or $R_3$-phenyl)-$C_1$-$C_7$-alkyl, wherein the hydroxy or amino group is separated from the ring-nitrogen atom by at least 2 carbon atoms ; and $R_2$ is hydrogen or $C_1$-$C_7$-alkyl ; $C_1$-$C_7$-alkanoyl, carbamoyl, mono- or di-$C_1$-$C_7$-alkylcarbamoyl derivatives of said (hydroxy or amino)-(phenyl or phenylene) compounds ; or salts thereof.

4. A process according to either of claims 1 or 2 for the preparation of a compound of the formula III

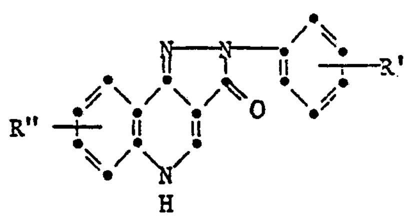

(III)

wherein R" is hydrogen, alkyl or alkoxy, each containing 4 carbon atoms, hydroxy, fluorine, chlorine, bromine or trifluoromethyl ; and R' is hydrogen, alkyl or alkoxy each containing 4 carbon atoms, hydroxy, fluorine, chlorine, bromine, trifluoromethyl, nitro, amino/monoalkylamino or alkylcarbamoylamino, each containing 4 carbon atoms, or cyano ; or a salt thereof.

5. A process according to claim 1 for the preparation of 2-(p-chlorophenyl)-pyrazolo [4,3-c] quinolin-3(5H)-one or a salt thereof.

6. A process according to claim 1 for the preparation of 2-phenyl-pyrazolo [4,3-c] quinolin-3(5H)-one or a salt thereof.

7. A process according to claim 1 for the preparation of a 2-aryl-pyrazolo [4,3-c] quinolin-3(1 or 5H)-one of the formula I and II as indicated in claim 1, wherein $R_3$ is hydrogen or not more than 3 identical or different members selected from $C_1$-$C_7$-alkyl, $C_1$-$C_7$-alkoxy, $C_1$-$C_7$-alkylthio, hydroxy, halogen, trifluoromethyl, nitro, amino, mono- or di-$C_1$-$C_7$-alkylamino, cyano, carbamoyl and carboxy ; R is phenyl or phenyl substituted by not more than 3 of the radicals as defined for $R_3$, pyridyl, $C_1$-$C_7$-alkylpyridyl, or halopyridyl ; $R_1$ is hydrogen, $C_1$-$C_7$-alkyl or (hydroxy, di-$C_1$-$C_7$-alkylamino or $R_3$-phenyl)-$C_1$-$C_7$-alkyl, wherein the hydroxy or amino group is separated from the ring nitrogen atom by at least 2 carbon atoms, and $R_2$ is hydrogen or $C_1$-$C_7$-alkyl ; their 3-hydroxy-tautomers ; $C_1$-$C_7$-alkanoyl, carbamoyl, mono- or di-$C_1$-$C_7$-alkylcarbamoyl derivatives of said (hydroxy or amino)-(phenyl or phenylene) compounds ; or salts thereof, which comprises

a) cyclising a compound of the formula IV

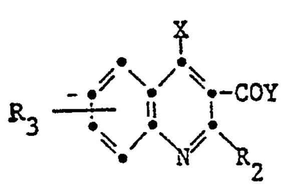

(IV)

wherein X is —NH—NH—R and Y is hydroxy or lower alkoxy ; or X is halogen and Y is $H_2\overset{|}{N}$—R, and, if desired, reacting a resulting compound, or an alkali metal salt thereof, with a reactive ester of the alcohol $R_1$—OH, or

b) condensing a compound of the formula V

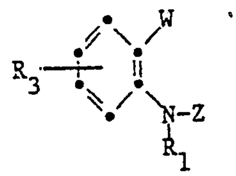

(V)

wherein W is

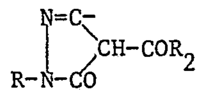

and Z is hydrogen, and, if desired, converting a resulting compound into another compound of the invention, and/or, if desired, converting a resulting compound into a salt thereof, or a resulting salt into the corresponding free compound or into another salt, and/or, if desired, separating a mixture of isomers obtained into the individual isomers.

8. A process according to claim 7, which comprises converting a resultant compound into another compound of the invention by substituting a compound, in which $R_1$ is hydrogen, in the 1-position by reaction with a reactive ester of the alcohol $R_1$—OH, and/or substituting a resultant compound of the formula II, in which $R_1$ is an alkali metal atom, in the 5-position by reaction with a reactive ester of the alcohol $R_1$—OH, and/or hydrolysing a resultant $C_1$-$C_7$-alkoxy compound to the corresponding phenol, and/or reducing a resultant nitro compound to the corresponding amine, and/or alkylating or acylating a resultant amino compound in which the acyl groups are as indicated in claim 7.

9. A process according to either of claims 7 or 8 for the preparation of a compound of the formula I and II as indicated in claim 1, wherein $R_3$ is hydrogen or one or two members selected from $C_1$-$C_7$-alkyl, $C_1$-$C_7$-alkoxy, $C_1$-$C_7$-alkylthio, hydroxy, halogen, trifluoromethyl, nitro, amino, mono- or di-$C_1$-$C_7$-alkylamino, cyano, carbamoyl and carboxy ; R is phenyl or phenyl substituted by one or two of the substituents as defined for $R_3$, pyridyl, $C_1$-$C_7$-alkylpyridyl or halopyridyl ; $R_1$ is hydrogen, $C_1$-$C_7$-alkyl or (hydroxy, di-$C_1$-$C_7$-alkylamino or $R_3$-phenyl)-$C_1$-$C_7$-alkyl, wherein the hydroxy or amino group is separated from the ring-nitrogen atom by at least 2 carbon atoms ; and $R_2$ is hydrogen or $C_1$-$C_7$-alkyl, or $C_1$-$C_7$-alkanoyl, carbamoyl, a mono- or di-$C_1$-$C_7$-alkylcarbamoyl derivative of the cited (hydroxy or amino)-(phenyl or phenylene) compounds, or a salt thereof.

10. A process according to either of claims 7 or 8 for the preparation of a compound of the formula III as indicated in claim 4, wherein R″ is hydrogen, alkyl or alkoxy, each containing 4 carbon atoms, hydroxy, fluorine, chlorine, bromine or trifluoromethyl, R′ is hydrogen, o- or m-fluorine, or a salt thereof.

11. A process according to claim 7 for the preparation of 2-(p-chlorophenyl)-pyrazolo [4,3-c] quinoline-3(5H)-one or a salt thereof.

12. A process for the manufacture of a pharmaceutical preparation, which comprises mixing a compound according to any one of claims 1 to 6 with a pharmaceutical carrier.

13. A process for the manufacture of a pharmaceutical preparation, which comprises mixing a compound according to any one of claims 7 to 10 with a pharmaceutical carrier.

**Revendications** (pour les Etats contractants : BE, CH, DE, FR, GB, IT, LI, LU, NL, SE)

1. Composés de formules générales I et II

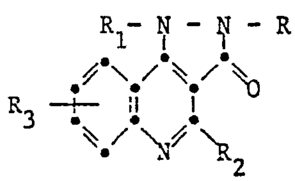

(I) et

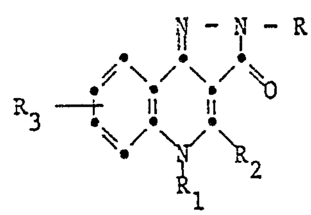

(II)

où $R_3$ représente un hydrogène ou au plus 3 substituants semblables ou différents du groupe alcoyle en $C_1$ à $C_7$, alcoxy en $C_1$ à $C_7$, alcoylthio en $C_1$ à $C_7$, hydroxy, halogène, trifluorométhyle, nitro, amino, mono- ou dialcoyle en $C_1$ à $C_7$-amino, cyano, carbamoyle ou carboxy, R représente un phényle non substitué ou substitué par au plus 3 des radicaux mentionnés pour $R_3$, un pyridyle, un alcoylpyridyle en $C_1$ à $C_7$ ou un halogéno-pyridyle, $R_1$ représente un hydrogène, un alcoyle en $C_1$ à $C_7$ ou un (hydroxy, di-alcoyle en $C_1$ à $C_7$-amino ou $R_3$-phényl)-alcoyle en $C_1$ à $C_7$, où le groupe hydroxy ou amino est séparé de

l'atome d'azote du noyau par au moins deux atomes de carbone, et $R_2$ représente un hydrogène ou un alcoyle en $C_1$ à $C_7$ ; leurs composés tautomères 3-hydroxy ; les dérivés alcanoyle en $C_1$ à $C_7$, carbamoyle, mono- ou di-alcoyle en $C_1$ à $C_7$-carbamoyle des composés (hydroxy ou amino)-(phényle ou phénylène) mentionnés ; ou leurs sels.

2. Composés selon la revendication 1, de formules I et II, où $R_3$ représente un hydrogène ou au plus 3 substituants semblables ou différents du groupe alcoyle en $C_1$ à $C_7$, alcoxy en $C_1$ à $C_7$, alcoylthio en $C_1$ à $C_7$, hydroxy, halogène, trifluorométhyle, nitro, amino, mono- ou dialcoyle en $C_1$ à $C_7$-amino, et les autres symboles ont les significations données dans la revendication 1.

3. Composés selon la revendication 1 de formules I et II, où $R_3$ représente un hydrogène ou un ou deux substituants du groupe alcoyle en $C_1$ à $C_7$, alcoxy en $C_1$ à $C_7$, alcoylthio en $C_1$ à $C_7$, hydroxy, halogène, trifluorométhyle, nitro, amino, mono- ou di-alcoyle en $C_1$ à $C_7$-amino, cyano, carbamoyle ou carboxy, R représente un phényle non substitué ou substitué par 1 ou 2 des radicaux donnés pour $R_3$, un pyridyle, un alcoyle en $C_1$ à $C_7$-pyridyle ou un halogénopyridyle, $R_1$ représente un hydrogène, un alcoyle en $C_1$ à $C_7$ ou un (hydroxy, di-alcoyle en $C_1$ à $C_7$-amino ou $R_3$-phényl)-alcoyle en $C_1$ à $C_7$, où le groupe hydroxy ou amino est séparé de l'atome d'azote du noyau par au moins deux atomes de carbone, et $R_2$ représente un hydrogène ou un alcoyle en $C_1$ à $C_7$.

4. Composés selon la revendication 1 de formules I et II, où $R_3$ représente un hydrogène ou un ou deux substituants du groupe alcoyle inférieur, alcoxy inférieur, alcoyle inférieur-thio, hydroxy, halogène, trifluorométhyle, nitro, amino, un mono- ou un di-alcoyle inférieur-amino et les autres symboles ont les significations données dans la revendication 3.

5. Composés selon la revendication 1, qui correspondent à la formule III

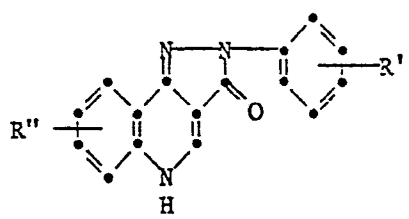

(III)

où R″ représente un hydrogène, un alcoyle ou un alcoxy avec à chaque fois 4 atomes de carbone, un hydroxy, un fluor, un chlore, un brome ou un trifluorométhyle, R′ représente un hydrogène, un alcoyle ou un alcoxy avec à chaque fois 4 atomes de carbone, un hydroxy, un fluor, un chlore, un brome, un trifluorométhyle, un nitro, un amino, un monoalcoylamino ou un alcoylcarbamoylamino avec chacun au plus 4 atomes de carbone, ou un cyano, ou leurs sels.

6. Composés selon la revendication 1 qui correspondent à la formule III présentée dans la revendication 5, où R″ représente un hydrogène, un alcoyle ou un alcoxy avec à chaque fois 4 atomes de carbone, un hydroxy, un fluor, un chlore, un brome ou un trifluorométhyle, R′ représente un hydrogène, un o- ou un m-fluor ; ou leurs sels.

7. La 2-(p-chlorophényl)-pyrazolo [4,3-c] quinolin-3(5H)-one ou ses sels.

8. La 2-phényl-pyrazolo [4,3-c] quinolin-3(5H)-one ou ses sels.

9. Composés selon la revendication 1, aux fins d'application comme substances actives psychotropes.

10. Composés selon la revendication 2, aux fins d'application comme substances actives psychotropes.

11. Préparations pharmaceutiques contenant des composés selon l'une des revendications 1, 3, 5, 8 et 9 ou des sels thérapeutiquement acceptables de tels composés.

12. Préparations pharmaceutiques contenant des composés selon l'une des revendications 2, 4, 6, 7 et 10 ou des sels thérapeutiquement acceptables de tels composés.

13. Les composés des revendications 1, 3, 5, 8 et 9 aux fins d'application comme agents psychotropes dans le traitement du corps humain ou animal.

14. Les composés des revendications 2, 4, 6, 7 et 10 aux fins d'application comme agents psychotropes dans le traitement du corps humain ou animal.

15. Application de composés des revendications 1, 3, 5, 8 et 9 aux fins de préparation de préparations pharmaceutiques.

16. Application de composés des revendications 2, 4, 6, 7 et 10 à la préparation de préparations pharmaceutiques.

17. Procédé de préparation de composés de 2-aryl-pyrazolo-[4,3-c] quinolin-3(1 et 5H)-one définis dans la revendication 1, caractérisé en ce que

a) on cyclise des composés de formule IV

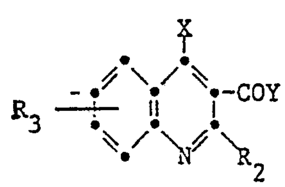

(IV)

où X représente —NH—NH—R et Y représente un hydroxy ou un alcoxy en $C_1$ à $C_7$ ; ou X représente un halogène et Y représente $H_2N—N—R$ ; ou bien où X représente un alcoxy en $C_1$ à $C_7$-amino ou un azido et Y représente NH—R, et, si on le désire, on fait réagir un composé obtenu ou un de ses sels de métaux alcalins avec un ester réactif d'un alcool $R_1$—OH, ou

b) on condense des composés de formule V

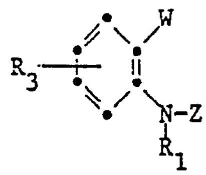

(V)

où les deux symboles W et $R_1$ représentent un hydrogène et Z représente le groupement

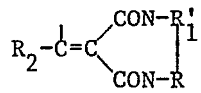

où $R_1'$ représente un alcoyle en $C_1$ à $C_7$ ; ou W est le groupement

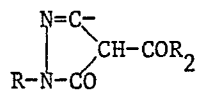

et Z représente un hydrogène ; ou W représente le groupement

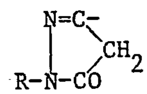

et Z représente $R_2$—CO ou $R_1$-N—Z pris ensemble représentent un isocyano et, si on le désire, on transforme un composé obtenu en un autre composé de l'invention, et/ou, si on le désire, on transforme un composé obtenu en un sel ou un sel obtenu en le composé libre ou en un autre sel, et/ou, si on le désire, on sépare un mélange d'isomères obtenu en les isomères isolés.

18. Les composés que l'on peut obtenir selon le procédé de l'exemple 17.

**Revendications** (pour l'Etat Contractant AT)

1. Procédé de préparation de nouveaux composés de 2-aryl-pyrazolo [4,3-c] quinolin-3 (1 et 5H)-one de formules générales I et II

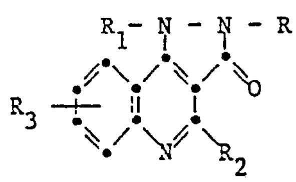

(I) et

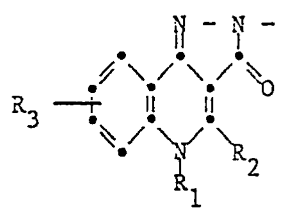

(II)

où $R_3$ représente un hydrogène ou au plus 3 substituants semblables ou différents du groupe alcoyle en $C_1$ à $C_7$, alcoxy en $C_1$ à $C_7$, alcoylthio en $C_1$ à $C_7$, hydroxy, halogène, trifluorométhyle, nitro, amino, mono- ou di-alcoyle en $C_1$ à $C_7$-amino, cyano, carbamoyle ou carboxy, R représente un phényle non substitué ou substitué par au plus 3 des radicaux mentionnés pour $R_3$, un pyridyle, un alcoylpyridyle en $C_1$ à $C_7$ ou un halogéno-pyridyle, $R_1$ représente un hydrogène, un alcoyle en $C_1$ à $C_7$ ou un (hydroxy, di-alcoyle en $C_1$ à $C_7$-amino ou $R_3$-phényl)-alcoyle en $C_1$ à $C_7$, où le groupe hydroxy ou amino est séparé de l'atome d'azote du noyau par au moins deux atomes de carbone, et $R_2$ représente un hydrogène ou un alcoyle en $C_1$ à $C_7$ ; leurs composés tautomères 3-hydroxy ; les dérivés alcanoyle en $C_1$ à $C_7$, carbamoyle, mono- ou di-alcoyle en $C_1$ à $C_7$-carbamoyle des composés (hydroxy ou amino)-(phényle ou phénylène) mentionnés ; ou leurs sels, caractérisé en ce que

a) on cyclise des composés de formule IV

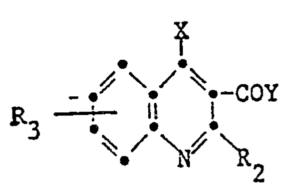

(IV)

où X représente —NH—NH—R et Y représente un hydroxy ou un alcoxy en $C_1$ à $C_7$ ; ou X représente un halogène et Y représente $H_2N$—N—R ; ou bien où X représente un alcoxy en $C_1$ à $C_7$-amino ou un azido et Y représente NH—R, et, si on le désire, on fait réagir un composé obtenu ou un de ses sels de métaux alcalins avec un ester réactif d'un alcool $R_1$—OH, ou

b) on condense des composés de formule V

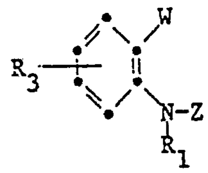

(V)

où les deux symboles W et $R_1$ représentent un hydrogène et Z représente le groupement

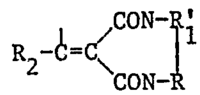

où $R_1'$ représente un alcoyle en $C_1$ à $C_7$ ; ou W est le groupement

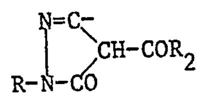

et Z représente un hydrogène ; ou W représente le groupement

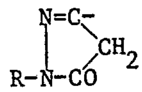

et Z représente $R_2$—CO ou $R_1$—N—Z pris ensemble représentent un isocyano et, si on le désire, on transforme un composé obtenu en un autre composé de l'invention, et/ou, si on le désire, on transforme un composé obtenu en un sel ou un sel obtenu en le composé libre ou en un autre sel, et/ou, si on le désire, on sépare un mélange d'isomères obtenu en les isomères isolés.

2. Procédé selon la revendication 1, caractérisé en ce qu'on transforme un composé obtenu en un autre composé de l'invention, en substituant un composé où $R_1$ représente un hydrogène, en position 1 par réaction avec un ester réactif de l'alcool $R_1$—OH, et/ou en substituant un composé de formule II obtenu, où $R_1$ représente un atome de métal alcalin, en poisition 5 par réaction avec un ester réactif de l'alcool $R_1$—OH, et/ou en hydrolysant un composé alcoxy en $C_1$ à $C_7$ obtenu pour donner le phénol correspondant, et/ou en réduisant un composé nitro obtenu pour donner l'amine correspondante, et/ou en alcoylant ou en acylant un composé amino obtenu, où les groupes acyle sont donnés dans la revendication 1, et/ou en transformant un nitrile obtenu en l'amide correspondant ou en l'hydrolysant pour donner le groupe carboxy.

3. Procédé selon l'une des revendications 1 et 2, caractérisé en ce qu'on prépare des composés de formules I et II données dans la revendication 1, où $R_3$ représente un hydrogène ou un ou deux substituants du groupe alcoyle en $C_1$ à $C_7$, alcoxy en $C_1$ à $C_7$, alcoylthio en $C_1$ à $C_7$, hydroxy, halogène, trifluorométhyle, nitro, amino, mono- ou di-alcoyle en $C_1$ à $C_7$-amino, cyano, carbamoyle ou carboxy, R représente un phényle non substitué ou substitué par 1 ou 2 des radicaux donnés pour $R_3$, un pyridyle, un alcoyle en $C_1$ à $C_7$-pyridyle ou un halogénopyridyle, $R_1$ représente un hydrogène, un alcoyle en $C_1$ à $C_7$ ou un (hydroxy, di-alcoyle en $C_1$ à $C_7$-amino ou $R_3$-phényl)-alcoyle en $C_1$ à $C_7$, où le groupe hydroxy ou amino est séparé de l'atome d'azote du noyau par au moins deux atomes de carbone, et $R_2$ représente un hydrogène ou un alcoyle en $C_1$ à $C_7$ ; les dérivés alcanoyle en $C_1$ à $C_7$, carbamoyle, mono- ou di-alcoyle en $C_1$ à $C_7$-carbamoyle des composés (hydroxy ou amino)-(phényle ou phénylène) mentionnés ; ou leurs sels.

4. Procédé selon l'une des revendications 1 et 2, caractérisé en ce qu'on prépare des composés de formule III

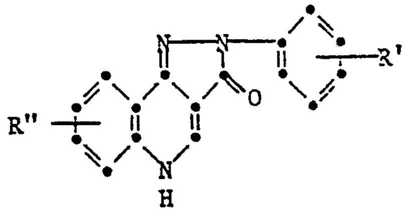

(III)

où R" représente un hydrogène, un alcoyle ou un alcoxy avec à chaque fois 4 atomes de carbone, un hydroxy, un fluor, un chlore, un brome ou un trifluorométhyle, R' représente un hydrogène, un alcoyle ou un alcoxy avec à chaque fois 4 atomes de carbone, un hydroxy, un fluor, un chlore, un brome, un trifluorométhyle, un nitro, un amino, un monoalcoylamino ou un alcoylcarbamoylamino avec chacun au plus 4 atomes de carbone, ou un cyano, ou leurs sels.

5. Procédé selon la revendication 1, caractérisé en ce qu'on prépare la 2-(p-chlorphényl)-pyrazolo [4,3-c] quinolin-3(5H)-one ou ses sels.

6. Procédé selon la revendication 1, caractérisé en ce qu'on prépare la 2-phényl-pyrazolo [4,3-c] quinolin-3(5H)-one ou ses sels.

7. Procédé selon la revendication 1 de préparation de composés de 2-aryl-pyrazolo [4,3-c] quinolin-3(1 et 5H)-one de formules I et II présentées dans la revendication 1, où $R_3$ représente un hydrogène ou au plus 3 substituants semblables ou différents du groupe alcoyle en $C_1$ à $C_7$, alcoxy en $C_1$ à $C_7$, alcoylthio en $C_1$ à $C_7$, hydroxy, halogène, trifluorométhyle, nitro, amino, mono- ou di-alcoyle en $C_1$ à $C_7$-amino, R représente un phényle non substitué ou substitué par au plus 3 des radicaux indiqués pour $R_3$, un pyridyle, un alcoyle en $C_1$ à $C_7$-pyridyle ou un halogéno-pyridyle, $R_1$ représente un hydrogène, un alcoyle en $C_1$ à $C_7$ ou un (hydroxy, di-alcoyle en $C_1$ à $C_7$-amino ou $R_3$-phényl)-alcoyle en $C_1$ à $C_7$, où le groupe hydroxy ou amino est séparé de l'atome d'azote du noyau par au moins deux atomes de carbone, et $R_2$ représente un hydrogène ou un alcoyle en $C_1$ à $C_7$ ; ainsi que de leurs composés tautomères 3-hydroxy ; et des dérivés alcanoyle en $C_1$ à $C_7$, carbamoyle, mono- ou di-alcoyle en $C_1$ à $C_7$-carbamoyle des composés (hydroxy ou amino)-(phényle ou phénylène) mentionnés ; ou de leurs sels, caractérisé en ce que

a) on cyclise des composés de formule IV

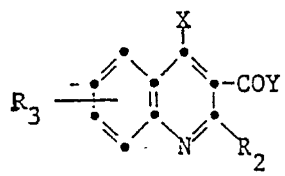

(IV)

où X représente —NH—NH—R et Y représente un hydroxy ou un alcoxy ifnérieur ; ou X représente un halogène et Y représente $H_2N—N—R$, et, si on le désire, on fait réagir un composé obtenu ou un de ses sels de métaux alcalins, avec un ester réactif d'un alcool $R_1$—OH, ou

b) on condense des composés de formule V

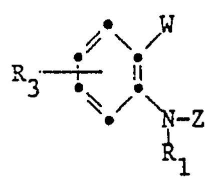

(V)

où W est le groupement

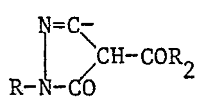

et, si on le désire, on transforme un composé obtenu en un autre composé de l'invention, et/ou, si on le désire, on transforme un composé obtenu en son sel ou un sel obtenu en le composé libre ou en un autre sel, et/ou, si on le désire, on dédouble un mélange d'isomères obtenu pour donner les isomères isolés.

8. Procédé selon la revendication 7, caractérisé en ce qu'on transforme un composé obtenu en un autre composé de l'invention, en substituant un composé où $R_1$ représente un hydrogène, en position 1 par réaction avec un ester réactif de l'alcool $R_1$—OH, et/ou en ce qu'on substitue un composé de formule II obtenu, où $R_1$ représente un atome de métal alcalin, en position 5 par réaction avec un ester réactif de l'alcool $R_1$-OH, et/ou en ce qu'on hydrolyse un composé alcoxy en $C_1$ à $C_7$ obtenu pour donner le phénol correspondant, et/ou en ce qu'on réduit un composé nitro obtenu pour donner l'amine correspondante, et/ou en ce qu'on alcoyle ou acyle un composé amino obtenu, où les groupes acyle sont donnés dans la revendication 7.

9. Procédé selon l'une des revendications 7 et 8, caractérisé en ce qu'on prépare des composés de formules I et II données dans la revendication 1, où $R_3$ représente un hydrogène ou un ou deux substituants du groupe alcoyle en $C_1$ à $C_7$, alcoxy en $C_1$ à $C_7$, alcoylthio en $C_1$ à $C_7$, hydroxy, halogène, trifluorométhyle, nitro, amino, mono- ou di-alcoyle en $C_1$ à $C_7$-amino, R représente un phényle non substitué ou substitué par un ou deux des radicaux donnés pour $R_3$, un pyridyle, un alcoyle en $C_1$ à $C_7$-pyridyle ou un halogénopyridyle, $R_1$ représente un hydrogène, un alcoyle en $C_1$ à $C_7$ ou un (hydroxy, di-

alcoyle en $C_1$ à $C_7$-amino ou $R_3$-phényl)-alcoyle en $C_1$ à $C_7$, où le groupe hydroxy ou amino est séparé de l'atome d'azote du noyau par au moins deux atomes de carbone, et $R_2$ représente un hydrogène ou un alcoyle en $C_1$ à $C_7$ ; les dérivés alcanoyle en $C_1$ à $C_7$, carbamoyle, mono- ou di-alcoyle en $C_1$ à $C_7$-carbamoyle des composés (hydroxy ou amino)-(phényle ou phénylène) mentionnés ; ou leurs sels.

10. Procédé selon l'une des revendications 7 et 8, caractérisé en ce qu'on prépare des composés de formule III présentée dans la revendication 4, où R'' représente un hydrogène, un alcoyle ou un alcoxy avec à chaque fois 4 atomes de carbone, un hydroxy, un fluor, un chlore, un brome ou un trifluorométhyle, R' représente un hydrogène, un o- ou un m-fluor ; ou leurs sels.

11. Procédé selon la revendication 7, caractérisé en ce qu'on prépare la 2-(p-chlorophényl)-pyrazolo [4,3-c]-quinolin-3(5H)-one ou ses sels.

12. Procédé de préparation d'une préparation pharmaceutique, caractérisé par le traitement d'une substance active selon l'invention selon l'une des revendications 1 à 6, avec un support pharmaceutique.

13. Procédé de préparation d'une préparation pharmaceutique, caractérisé par le traitement d'une substance active selon l'invention selon l'une des revendications 7 à 10, avec un support pharmaceutique.